(19) Europäisches Patentamt
European Patent Office
Office européen des brevets

(11) EP 1 100 778 B1

(12) **EUROPEAN PATENT SPECIFICATION**

(45) Date of publication and mention of the grant of the patent:
**01.12.2004 Bulletin 2004/49**

(21) Application number: **99941465.9**

(22) Date of filing: **21.07.1999**

(51) Int Cl.[7]: **C07D 207/34**, A61K 31/40

(86) International application number:
**PCT/EP1999/005349**

(87) International publication number:
**WO 2000/006542 (10.02.2000 Gazette 2000/06)**

(54) **OXIDISED SULFURATED DISTAMYCIN DERIVATIVES, PROCESS FOR PREPARING THEM, AND THEIR USE AS ANTITUMOR AGENTS**

OXIDIERTE SULFURIERTE DISTAMYCINDERIVATIVE, VERFAHREN ZU IHRER HERSTELLUNG UND IHRE VERWENDUNG ALS ANTITUMORMITTEL

DERIVES DISTAMYCINE SULFURES ET OXYDES, LEUR PROCEDE DE PREPARATION ET UTILISATION EN TANT QU'AGENTS ANTITUMORAUX

(84) Designated Contracting States:
**DE GB IT**

(30) Priority: **30.07.1998 GB 9816653**

(43) Date of publication of application:
**23.05.2001 Bulletin 2001/21**

(73) Proprietor: **Pharmacia Italia S.p.A.**
**20152 Milano (IT)**

(72) Inventors:
- **COZZI, Paolo**
  **I-20133 Milan (IT)**
- **CALDARELLI, Marina**
  **I-20147 Milan (IT)**
- **BERIA, Italo**
  **I-45030 Villamarzana (IT)**
- **GERONI, Maria, Cristina**
  **I-20149 Milan (IT)**
- **CAPOLONGO, Laura**
  **I-20147 Milan (IT)**

(74) Representative: **Modiano, Micaela Nadia et al**
**Modiano, Josif, Pisanty & Staub,**
**Baaderstrasse 3**
**80469 München (DE)**

(56) References cited:
**EP-A- 0 246 868**
**WO-A-97/28123**
**WO-A-97/43258**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 100 778 B1

## Description

The present invention relates to new alkylating antitumor agents analogous to Distamycin A, to a process for their preparation, to pharmaceutical compositions containing them and to their use as therapeutic agents.

Distamycin A, whose formula is reported below

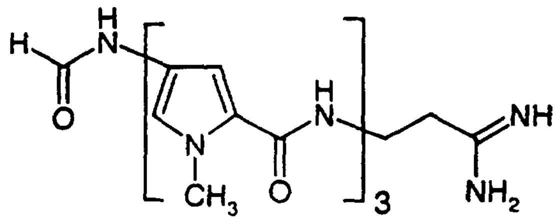

belongs to the family of the pyrroleamidine antibiotics and it is reported to interact reversibly and selectively with DNA-AT sequences, thus interfering with both replication and transcription. See, for a reference, Nature, 203, 1064 (1964); FEBS Letters, 7 (1970) 90; Prog. Nucleic Acids Res. Mol, Biol., 15, 285 (1975).

Several analogous to distamycin are known in the art.

DE-A-1795539 discloses distamycin derivatives in which the formyl group is replaced by a hydrogen atom or by the carboxylic acid residue of a $C_1$-$C_4$ aliphatic or cyclopentylpropionic acid.

EP-A-246,868 describes distamycin analogues in which the distamycin formyl group is substituted by aromatic, alicyclic or heterocyclic moieties bearing alkylating groups.

WO 97/28123 and WO 97/43258 describe distamycin analogues in which the amidino group is replaced with different nitrogen-containing ending groups and the distamycin formyl group is substituted by an aromatic or a cinnamoyl moiety, respectively.

It has now been found that a new class of distamycin derivatives as defined hereinunder, wherein the distamycin formyl group is substituted by a phenylcarbonyl, phenylalkylcarbonyl or phenylalkenylcarbonyl group bearing a haloethyl-sulfinyl or a haloethyl-sulfonyl group as an alkylating moiety, and the amidino group is optionally replaced by various nitrogen-containing ending groups, shows valuable biological properties.

Therefore, the present invention provides compounds which are oxidised sulfurated distamycin derivatives of formula:

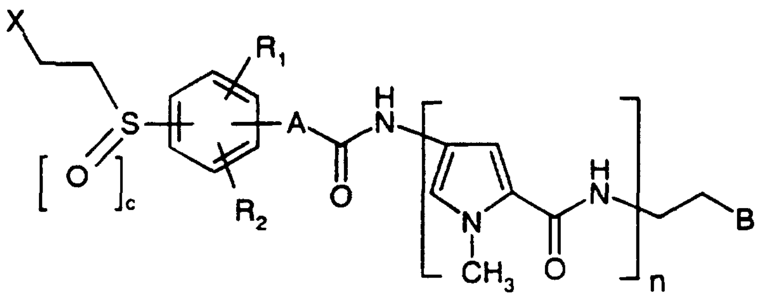

(I)

wherein:

n is 2, 3 or 4;
c is 1 or 2;
A is a bond, a $C_1$-$C_4$ alkylene or $C_2$-$C_4$ alkenylene group;
$R_1$ and $R_2$, which are the same or different, are selected from hydrogen, $C_1$-$C_4$ alkyl optionally substituted by one or more fluorine atoms, and $C_1$-$C_4$ alkoxy;
X is a halogen atom;
B is selected from:

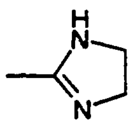 , 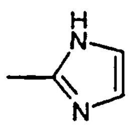 , 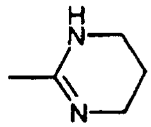 , 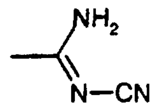 , 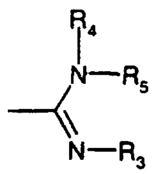 , 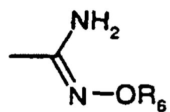 ,

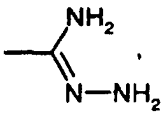 , —CN , 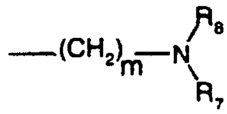 , 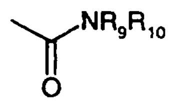 and 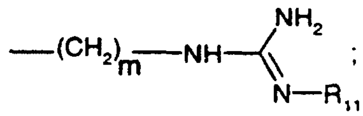 ;

wherein $R_3$, $R_4$, $R_5$, $R_6$, $R_7$, $R_8$, $R_9$ and $R_{10}$, which are the same or different, are selected from hydrogen or $C_1$-$C_4$ alkyl; $R_{11}$ is hydrogen, $C_1$-$C_4$ alkyl or hydroxy, and m is 0, 1 or 2;
or pharmaceutically acceptable salts thereof.

The present invention includes within its scope also all the possible isomers covered by the compounds of formula (I), both separately and in admixture, as well as the metabolites and the pharmaceutically acceptable bioprecursors (otherwise known as pro-drugs) of the compounds of formula (I).

In the present description, unless otherwise specified, both terms alkyl and alkoxy include straight or branched $C_1$-$C_4$ alkyl and alkoxy groups such as, for instance, methyl, ethyl, n-propyl, isopropyl, n-butyl, isobutyl, sec-butyl, tert-butyl, methoxy, ethoxy, n-propoxy, isopropoxy, n-butoxy, isobutoxy, sec-butoxy and tert-butoxy.

Preferred $C_1$-$C_4$ alkyl or alkoxy groups are methyl, ethyl, methoxy and ethoxy groups.

When substituted by one or more fluorine atoms, the $C_1$-$C_4$ alkyl groups are preferably $C_1$-$C_4$ perfluoroalkyl groups, e.g. trifluoromethyl.

Both terms alkylene and alkenylene refer, respectively, to $C_1$-$C_4$ alkylene or $C_2$-$C_4$ alkenylene groups, as bivalent radicals of the corresponding $C_1$-$C_4$ saturated or $C_2$-$C_4$ unsaturated hydrocarbons.

Preferred alkylene or alkenylene groups according to the present invention are methylene, ethylene or vinylene groups.

The term halogen atom includes fluorine, chlorine, bromine and iodine, being chlorine and bromine preferred.

Within the compounds of formula (I) the haloethyl-sulfinyl or sulfonyl group and the A group are in ortho, meta or para position with respect to each other; preferably, they are in meta or para position.

Pharmaceutically acceptable salts of the compounds of formula (I) are their salts with pharmaceutically acceptable either inorganic or organic acids such as, for instance, hydrochloric, hydrobromic, sulfuric, nitric, acetic, propionic, succinic, malonic, citric, tartaric, methanesulfonic and p-toluenesulfonic acid.

A preferred class of compounds of the present invention is that wherein, in formula (I):

n is 3;
c is 1;
A is a bond or vinylene;
$R_1$ and $R_2$ which are the same or different, are selected from hydrogen, methyl, methoxy or trifluoromethyl;
X is chloro or bromo;
B is selected from:

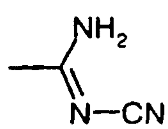 , 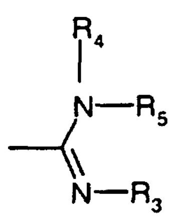 , 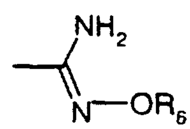 , —CN ,

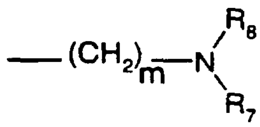 , 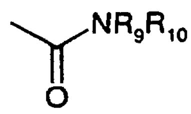 and 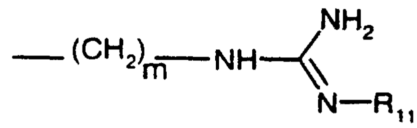

wherein $R_3$, $R_4$, $R_5$, $R_7$, $R_8$, $R_9$, $R_{10}$ and $R_{11}$, which are the same or different, are selected from hydrogen or methyl; $R_6$ is hydrogen; and m is 0 or 1;
or the pharmaceutically acceptable salts thereof.

Examples of specific compounds according to the present invention, especially in the form of salts, preferably with hydrochloric acid, are the following:

1)3-[1-methyl-4[1-methyl-4[1-methyl-4[4-(2-chloroethylsulfinyl)phenyl-1-carboxamido]pyrrole-2-carboxamido]pyrrole-2-carboxamido]pyrrole-2-carboxamido]propionamidine;
2)3-[1-methyl-4[1-methyl-4[1-methyl-4[4-(2-chloroethylsulfinyl)phenyl-1-carboxamido]pyrrole-2-carboxamido]pyrrole-2-carboxamido]pyrrole-2-carboxamido]propion-N-methylamidine;
3)3-[1-methyl-4[1-methyl-4[1-methyl-4[4-(2-chloroethylsulfinyl)phenyl-1-carboxamido]pyrrole-2-carboxamido]pyrrole-2-carboxamido]pyrrole-2-carboxamido]propion-N,N'-dimethylamidine;
4)3-[1-methyl-4[1-methyl-4[2-methyl-4[4-(2-chloroethylsulfinyl)phenyl-1-carboxamido]pyrrole-2-carboxamido]pyrrole-2-carboxamido]pyrrole-2-carboxamido]propion-N,N',N'-trimethylamidine;
5)3-[1-methyl-4[1-methyl-4[1-methyl-4[4-(2-chloroethylsulfinyl)phenyl-1-carboxamido]pyrrole-2-carboxamido]pyrrole-2-carboxamido]pyrrole-2-carboxamido]propion-N-cyanamidine;
6)3-[1-methyl-4[1-methyl-4[1-methyl-4[4-(2-chloroethylsulfinyl)phenyl-1-carboxamido]pyrrole-2-carboxamido]pyrrole-2-carboxamido]pyrrole-2-carboxamido]propionamidoxime;
7)3-[1-methyl-4[1-methyl-4[1-methyl-4[4-(2-chloroethylsulfinyl)phenyl-1-carboxamido]pyrrole-2-carboxamido)pyrrole-2-carboxamido]pyrrole-2-carboxamido]propionamide;
8)3-[1-methyl-4[1-methyl-4[1-methyl-4[4-(2-chloroethylsulfinyl)phenyl-1-carboxamido]pyrrole-2-carboxamido]pyrrole-2-carboxamido]pyrrole-2-carboxamido]propion-N-methylamide;
9)3-[1-methyl-4[1-methyl-4[1-methyl-4[4-(2-chloroethylsulfinyl)phenyl-1-carboxamido]pyrrole-2-carboxamido]pyrrole-2-carboxamido]pyrrole-2-carboxamido]propionitrile;
10)2-[1-methyl-4[1-methyl-4[1-methyl-4[4-(2-chloroethylsulfinyl)phenyl-1-carboxamido]pyrrole-2-carboxamido]pyrrole-2-carboxamido]pyrrole-2-carboxamido]ethylguanidine;
11)3-[1-methyl-4[1-methyl-4[1-methyl-4[4-(2-chloroethylsulfinyl)phenyl-1-carboxamido]pyrrole-2-carboxamido]pyrrole-2-carboxamido]pyrrole-2-carboxamido]propion-N,N-dimethylamine;
12)3-[1-methyl-4[1-methyl-4[1-methyl-4[4-(2-bromoethylsulfinyl)phenyl-1-carboxamido]pyrrole-2-carboxamido]pyrrole-2-carboxamido]pyrrole-2-carboxamido]propionamidine;
13)3-[1-methyl-4[1-methyl-4[1-methyl-4[3-methyl-4-(2-chloroethylsulfinyl)phenyl-1-carboxamido]pyrrole-2-carboxamido]pyrrole-2-carboxamido]pyrrole-2-carboxamido]propionamidine;
14)3-[1-methyl-4[1-methyl-4[1-methyl-4[4-(2-chloroethylsulfinyl)cinnamoyl-1-carboxamido]pyrrole-2-carboxamido]pyrrole-2-carboxamido]pyrrole-2-carboxamido]propionamidine;
15)3-[1-methyl-4[1-methyl-4[1-methyl-4[4-(2-chloroethylsulfinyl)cinnamoyl-1-carboxamido]pyrrole-2-carboxamido]pyrrole-2-carboxamido]pyrrole-2-carboxamido]propion-N-methylamidine;
16)3-[1-methyl-4[1-methyl-4[1-methyl-4[4-(2-chloroethylsulfinyl)cinnamoyl-1-carboxamido]pyrrole-2-carboxamido]pyrrole-2-carboxamido]pyrrole-2-carboxamido]propion-N,N'-dimethylamidine;
17)3-[1-methyl-4[1-methyl-4[1-methyl-4[4-(2-chloroethylsulfinyl)cinnamoyl-1-carboxamido]pyrrole-2-carboxamido]pyrrole-2-carboxamido]pyrrole-2-carboxamido]propion-N,N,-dimethylamidine;
18)3-[1-methyl-4[1-methyl-4[1-methyl-4[4-(2-chloroethylsulfinyl)cinnamoyl-1-carboxamido]pyrrole-2-carboxamido]pyrrole-2-carboxamido]pyrrole-2-carboxamido]propion-N-cyanamidine;
19)3-[1-methyl-4[1-methyl-4[1-methyl-4[4-(2-chloroethylsulfinyl)cinnamoyl-1-carboxamido]pyrrole-2-carboxamido]pyrrole-2-carboxamido]pyrrole-2-carboxamido]propionamidoxime;
20)3-[1-methyl-4[1-methyl-4[1-methyl-4[4-(2-chloroethylsulfinyl)cinnamoyl-1-carboxamido]pyrrole-2-carboxamido)pyrrole-2-carboxamido]pyrrole-2-carboxamido]propionamide;
21)3-[1-methyl-4[1-methyl-4[1-methyl-4[4-(2-chloroethylsulfinyl)cinnamoyl-1-carboxamido]pyrrole-2-carboxamido]pyrrole-2-carboxamido]pyrrole-2-carboxamido]propionamide;
22)3-[1-methyl-4[1-methyl-4[1-methyl-4[4-(2-chloroethylsulfinyl)cinnamoyl-1-carboxamido]pyrrole-2-carboxamido]pyrrole-2-carboxamido]pyrrole-2-carboxamido]propionitrile;
23)2-[1-methyl-4[1-methyl-4[1-methyl-4[4-(2-chloroethylsulfinyl)cinnamoyl-1-carboxamido]pyrrole-2-carboxamido]pyrrole-2-carboxamido]pyrrole-2-carboxamido]ethylguanidine;
24)3-[1-methyl-4[1-methyl-4[1-methyl-4[4-(2-chloroethylsulfinyl)cinnamoyl-1-carboxamido]pyrrole-2-carboxamido]pyrrole-2-carboxamido]pyrrole-2-carboxamido]propion-N,N,N'-trimethylamidine;
25)3-[1-methyl-4[1-methyl-4[1-methyl-4[4-(2-chloroethylsulfonyl)phenyl-1-carboxamido]pyrrole-2-carboxamido] pyrrole-2-carboxamido]pyrrole-2-carboxamido]propionamidine;
26)3-[1-methyl-4[1-methyl-4[1-methyl-4[4-(2-chloroethylsulfonyl)phenyl-1-carboxamido]pyrrole-2-carboxamido] pyrrole-2-carboxamido]pyrrole-2-carboxamido]propion-N-methylamidine;
27)3-[1-methyl-4[1-methyl-4[1-methyl-4[4-(2-chloroethylsulfonyl)phenyl-1-carboxamido]pyrrole-2-carboxamido] pyrrole-2-carboxamido]pyrrole-2-carboxamido]propion-N,N'-dimethylamidine;
28)3-[1-methyl-4[1-methyl-4[1-methyl-4[4-(2-chloroethylsulfonyl)phenyl-1-carboxamido]pyrrole-2-carboxamido] pyrrole-2-carboxamido]pyrrole-2-carboxamido]propion-N-cyanamidine;
29)3-[1-methyl-4[1-methyl-4[1-methyl-4[4-(2-chloroethylsulfonyl)phenyl-1-carboxamido]pyrrole-2-carboxamido] pyrrole-2-carboxamido]pyrrole-2-carboxamido]propionamidoxime;
30)3-[1-methyl-4[1-methyl-4[1-methyl-4[4-(2-chlaroethylsulfonyl)phenyl-1-carboxamido]pyrrole-2-carboxamido] pyrrole-2-carboxamido]pyrrole-2-carboxamido]propionamide;
31)3-[1-methyl-4[1-methyl-4[1-methyl-4[4-(2-chloroethylsulfonyl)phenyl-1-carboxamido]pyrrole-2-carboxamido] pyrrole-2-carboxamido]pyrrole-2-carboxamido]propionitrile;
32)2-[1-methyl-4[1-methyl-4[1-methyl-4[4-(2-chloroethylsulfonyl)phenyl-1-carboxamido]pyrrole-2-carboxamido] pyrrole-2-carboxamido]pyrrole-2-carboxamido]ethylguanidine;
33)3-[1-methyl-4[1-methyl-4[1-methyl-4[4-(2-chloroethylsulfonyl)cinnamoyl-1-carboxamido]pyrrole-2-carboxamido]pyrrole-2-carboxamido]pyrrole-2-carboxamido]propionamidine;
34)3-[1-methyl-4[1-methyl-4[1-methyl-4[4-(2-chloroethylsulfonyl)cinnamoyl-1-carboxamido]pyrrole-2-carboxamido]pyrrole-2-carboxamido]pyrrole-2-carboxamido]propion-N-methylamidine;
35)3-[1-methyl-4[1-methyl-4[1-methyl-4[4-(2-chloroethylsulfonyl)cinnamoyl-1-carboxamido]pyrrole-2-carboxamido]pyrrole-2-carboxamido]pyrrole-2-carboxamido]propion-N,N'-dimethylamidine;
36)3-[1-methyl-4[1-methyl-4[1-methyl-4[4-(2-chloroethylsulfonyl)cinnamoyl-1-carboxamido]pyrrole-2-carboxamido]pyrrole-2-carboxamido]pyrrole-2-carboxamido]propion-N-cyanamidine;
37)3-[1-methyl-4[1-methyl-4[1-methyl-4[4-(2-chloroethylsulfonyl)cinnamoyl-1-carboxamido]pyrrole-2-carboxamido]pyrrole-2-carboxamido]pyrrole-2-carboxamido]propionamidoxime;
38)3-[1-methyl-4[1-methyl-4[1-methyl-4[4-(2-chloroethylsulfonyl)cinnamoyl-1-carboxamido]pyrrole-2-carboxamido]pyrrole-2-carboxamido]pyrrole-2-carboxamido]propionamide;
39)3-[1-methyl-4[1-methyl-4[1-methyl-4[4-(2-chloroethylsulfonyl)cinnamoyl-1-carboxamido]pyrrole-2-carboxamido]pyrrole-2-carboxamido]pyrrole-2-carboxamido]propionitrile;
40)2-[1-methyl-4[1-methyl-4[1-methyl-4[4-(2-chloroethylsulfonyl)cinnamoyl-1-carboxamido]pyrrole-2-carboxamido]pyrrole-2-carboxamido]pyrrole-2-carboxamido]ethylguanidine.

A further object of the present invention is a process for preparing the compounds of formula (I), and the pharmaceutically acceptable salts thereof, which process comprises:

(a) when B is other than

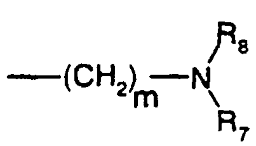

and

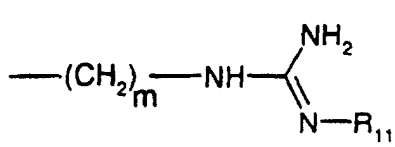

reacting a compound of formula:

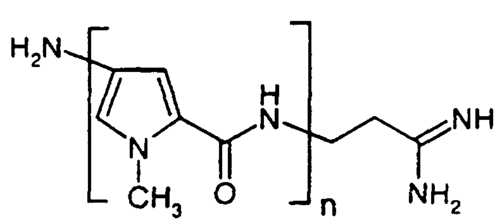

(II)

with a compound of formula:

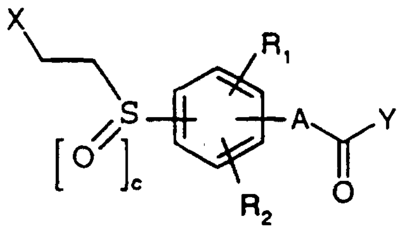 (III)

wherein n, c, $R_1$, $R_2$, X and A are as defined above, and Y is hydroxy or a suitable leaving group; so as to obtain a compound of formula:

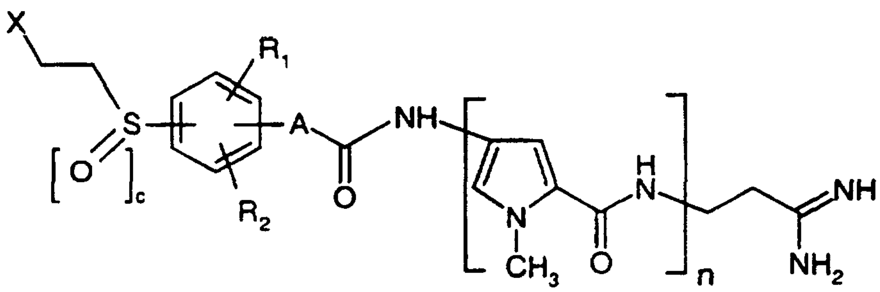 (Ia)

and, then, optionally reacting a compound of formula (Ia) with:

(i) $H_2N-(CH_2)_r-NH_2$, wherein r is 2 or 3, so as to obtain a compound of formula (I) having B equal to:

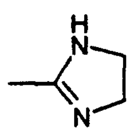 or 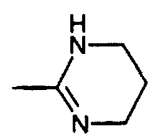

(ii) $H_2N-CH_2-CHO$, so obtaining a compound of formula (I) having B equal to:

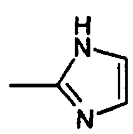

(iii)$H_2N-CN$, so obtaining a compound of formula (I) having B equal to:

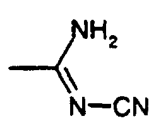

(iv) $H_2N-OR_6$, so obtaining a compound of formula (I) having B equal to:

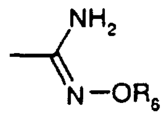

(v) $H_2N\text{-}NH_2$, so obtaining a compound of formula (I) having B equal to:

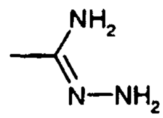

(vi) $HNR_4R_5$, so obtaining a compound of formula (I) having B equal to:

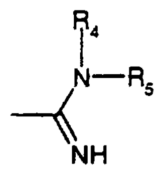

and then optionally with $H_2NR_3$, so obtaining a compound of formula (I) having B equal to:

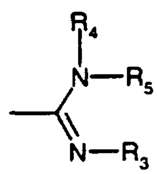

(vii)succinic anhydride, so obtaining a compound of formula (I) having B equal to -C≡N;
(viii)water in an alkaline medium, so obtaining a compound of formula (I) having B equal to $-CONR_9R_{10}$ wherein $R_9$ and $R_{10}$ are both hydrogen atoms;
(ix) $HNR_9R_{10}$, so obtaining a compound of formula (I) having B equal to:

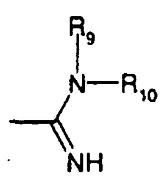

and then with water in an alkaline medium, so obtaining a compound of formula (I) having B equal to $-CONR_9R_{10}$, wherein $R_9$ and $R_{10}$ are, each independently, hydrogen or $C_1$-$C_4$ alkyl; or
(b) when B is other than

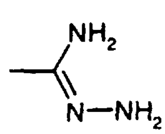

reacting a compound of formula:

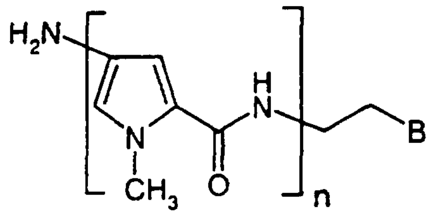

(IV)

with a compound of formula:

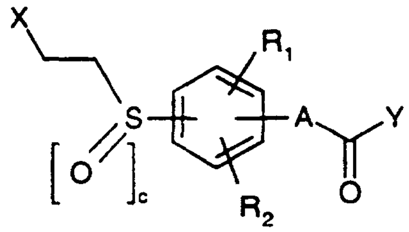

(III)

wherein n, c, B, $R_1$, $R_2$, X, Y and A are as defined above;
so obtaining the corresponding compound of formula (I);
and, if desired, converting the compound of formula (I) into a pharmaceutically acceptable salt thereof.

In formula (III), Y is hydroxy or a leaving group selected, for instance, from chloro, 2,4,5-trichlorophenoxy, 2,4-dinitro-phenoxy, succinimido-N-oxy, imidazolyl group, and the like.

The condensation reactions as set forth above under processes (a) and (b) is carried out according to known methods, for instance those described in the aforementioned EP-A-246,868.

The reaction between a compound of formula (II) or (IV) with a compound of formula (III) is preferably carried out with a molar ratio (II):(III) or (IV):(III) of from 1:1 to 1:2.

Within the compounds of formula (III) wherein Y is hydroxy, the reaction is carried out in an organic solvent, such as, dimethylsulphoxide, hexamethylphosphotriamide, dimethylacetamide, dimethylformamide, ethanol, phenyl, or pyridine, in the presence of an organic or inorganic base such as triethylamine, diisopropyl ethylamine, or sodium or potassium carbonate or bicarbonate, and of a condensing agent such as, N-ethyl-N'-(3-dimethylamino-propyl)-carbodiimide, N,N'-dicyclohexyl-carbodiimide, or 1-hydroxy-benzotriazole hydrate.

The reaction temperature may vary from about -10°C to about 100°C, and the reaction time from about 1 to about 24 hours.

Within the compounds of formula (III) wherein Y is a leaving group as set forth above, the aforementioned condensation reaction may be carried out in an organic solvent such as, for instance, dimethylformamide, dioxane, pyridine, tetrahydrofurane, or mixtures thereof with water, optionally in the presence of an organic or inorganic base, e.g. N,N'-diisopropylethylamine, triethylamine, sodium or potassium bicarbonate, at a temperature of from about 0°C to about 100°C, and for a time varying from about 2 hours to about 48 hours.

The reaction between a compound of formula (Ia) according to process (a) and one of the reactants as described above at points (i)-(vi) or (ix), can be carried out according to known methods, for instance those reported in US-4,766,142; WO 97/28123; Chem. Revs. 1961, 155; J. Med. Chem. 1984, 27, 849-857; Chem. Revs. 1970, 151; and "The Chemistry of Amidines and Imidates", edited by S. Patai, John Wiley & Sons, N.Y. (1975).

The reaction of a compound of formula (Ia) with succinic anhydride, as defined in point (vii) above, is preferably carried out with a molar ratio (Ia):succinic anhydride of from 1:1 to 1:3 in an organic solvent such as, for instance, dimethyl sulphoxide or dimethylformamide, and in the presence of an organic or inorganic base such as, e.g., triethylamine, diisopropylethylamine, sodium or potassium carbonate, and the like. The reaction temperature may vary from about 25°C to about 100°C, and the reaction time from about 1 hour to about 12 hours.

The reaction with water in an alkaline medium, as defined in points (viii) and (ix) above, may be carried out according to known methods usually employed for alkaline hydrolysis, for instance by treating the substrate with an excess of sodium or potassium hydroxide in water or in a water/organic solvent admixture, e.g. dioxane, tetrahydrofuran, or acetonitrile, at a temperature of from about 50°C to about 100°C, for a time varying from about 2 hours to about 48 hours.

The compounds of formula (II) are known or may be prepared according to known methods; see, for a reference, Arcamone et al. in Gazzetta Chim. Ital. 97, 1097 (1967).

Also the compounds of formula (III) are known or may be prepared according to known methods, for instance by working as described in J. Org. Chem. 1993, 58, 4506-4508; Helvetica Chimica Acta, Vol. 67,(1984), 1316-1327; Tetrahedron Letters 35, 3457-3460, 1994; J. Chem. Soc. Perkin Trans. 1, 2961, 1991.

The compounds of formula (IV) are known compounds as well, for instance as reported in the aforementioned WO 97/28123.

In view of what above reported, it is clear to the man skilled in the art that when preparing the compounds of formula (I) as set forth above, optional amino groups, i.e. $R_7$ and/or $R_8$ of the compounds of formula (IV) equal to hydrogen, need to be properly protected according to conventional techniques, so as to avoid unwanted side reactions.

Likewise, the conversion of the said protected amino groups into the free amines may be carried out according to known procedures. See, for a general reference, J. Org. Chem. 43, 2285, (1978); J. Org. Chem. 44, 811 (1979); J. Am. Chem. Soc. 78, 1359 (1956); Ber. 65, 1192 (1932); and J. Am Chem. Soc. 80, 1154, (1958).

Salification of a compound of formula (I), as well as preparation of a free compound starting from a salt, may be carried out by known standard methods.

Well known procedures such as, e.g., fractional crystallisation or chromatography, may also be followed for separating a mixture of isomers of formula (I) into the single isomers.

The compounds of formula (I) may be purified by conventional techniques such as, e.g., silica gel or alumina column chromatography, and/or by recrystallisation from an organic solvent such as, e.g., a lower aliphatic alcohol, e. g. methyl, ethyl or isopropyl alcohol, or dimethylformamide.

## PHARMACOLOGY

The compounds of formula (I) according to the present invention are useful as antineoplastic agents.

In particular, the interest in the development of these molecules (hypoxia-selective cytotoxic agents) is related to their effect against tumor cell populations which grow at very low oxygen concentrations in solid tumors and which appear to limit the effectiveness of conventional chemotherapy.

The antineoplastic activity of the compounds was evaluated in vivo against advanced human mammary carcinoma xenograft (MX-1) showing a very good antitumor activity.

MX-1 human mammary (originally obtained from NCI, NHI, Bethesda, MD) was transplanted s.c. in athymic mice using 15-20 mg of tumor brei. The tumor model was maintained in vivo in adult female Hsd:athymic nude mice.

Nude mice were 4-6 weeks old, weighed 20-25 g and were maintained in cages with paper filter covers; food and bedding were sterilised and water was acidified (pH 2.5-3). All animals were supplied by Harlan Nossan (Italy).

The mouse colony was routinely tested monthly for the absence of antibodies to a panel of pathogens including Mouse hepatitis, Sendai Virus and Mycoplasma pulmonis.

Drug activity was determined on advanced solid tumors (when tumor mass is > 500 mg); tumor growth was assessed by caliper measurement, and tumor weight was estimated according to Geran.

The antitumor effect was determined by comparing tumor weights in the treated group and those of the control group on a given day. The percentage of tumor growth inhibition (%T.I.) was calculated 7 days after the last treatment, according to the following equation:

100-(median tumor weight of treated group/median tumor weight of control group)x100

Tumor-free mice 90 days after tumor implant are considered cured mice.

Toxicity was evaluated on the basis of the body weight reduction and gross autopsy findings, mainly in terms of reduction of spleen and liver size.

All drug solutions were prepared immediately before use. Treatment was administered (q4dx4) intravenously in a volume of 10 ml/kg of body weight.

The compounds of the invention can be administered to mammals, including humans, through the usual routes, for example, parenterally, e.g. by intravenous injection or infusion, intramuscularly, subcutaneously, topically or orally. The dosage depends on age, weight and conditions of the patient and on the administration route. For example, a suitable dosage for administration to adult humans may range from about 0.1 to about 150-200 mg pro dose 1-4 times a day.

Further object of the present invention are pharmaceutical compositions, which comprise a compound of formula (I) as an active principle, in association with one or more pharmaceutically acceptable carrier and/or diluent.

The pharmaceutical compositions of the present invention are usually prepared following conventional methods and are administered in a pharmaceutically suitable form. For instance, solutions for intravenous injection or infusion may contain as a carrier, for example, sterile water or preferably, they may be in the form of sterile aqueous isotonic saline solutions.

Suspensions or solutions for intramuscular injections may contain, together with the active compound a pharmaceutically acceptable carrier, e.g. sterile water, olive oil, ethyl oleate, glycols, *e.g.* propylene glycol, and if desired, a suitable amount of lidocaine hydrochloride.

In the forms for topical application, e.g. creams, lotions or pastes for use in dermatological treatment, the active ingredient may be mixed with conventional oleaginous or emulsifying excipients.

The solid oral forms, e.g. tablets and capsules, may contain, together with the active compound, diluents, e.g., lactose, dextrose, saccharose, cellulose, corn starch and potato starch; lubricants, e.g. silica, talc, stearic acid, magnesium or calcium stearate, and/or polyethylene glycols; binding agents, e.g. starches, arabic gums, gelatin, methylcellulose, carboxymethyl cellulose, polyvinylpyrrolidone; disaggregating agents, e.g. starch, alginic acid, alginates, sodium starch glycolate; effervescing mixtures; dyestuffs; sweeteners; wetting agents, for instance, lecithin, polysorbates, laurylsulphates; and, in general, non-toxic and pharmacologically inactive substances used in pharmaceutical formulation. Said pharmaceutical preparations may be manufactured by known techniques, for example by means of mixing, granulating, tabletting, sugar-coating or film-coating processes.

Further object of the present invention are the compounds of formula (I) for use in a method for treating the human or animal body by therapy.

Furthermore, the present invention provides a method for treating tumors in a patient in need of it, which comprises administering to said patient a composition of the invention.

A further object of the present invention is a combined method for treating cancer or for ameliorating the conditions of mammals, including humans, suffering from cancer, said method comprising administering a compound of formula (I), or a pharmaceutically acceptable salt thereof, and an additional antitumor agent, close enough in time and in amounts sufficient to produce a therapeutically useful effect.

The present invention also provides products containing a compound of formula (I), or a pharmaceutically acceptable salt thereof, and an additional antitumour agent as a combined preparation for simultaneous, separate or sequential use in anti-cancer therapy.

The term "antitumor agent" is meant to comprise both a single antitumor drug and "cocktails" i.e. a mixture of such drugs, according to the clinical practice. Examples of antitumor agents that can be formulated with a compound of formula (I), or alternatively, can be administered in a combined method of treatment, include doxorubicin, daunomycin, epirubicin, idarubicin, etoposide, fluorouracil, melphalan, cyclophosphamide, 4-demethoxy daunorubicin, bleomycin, vinblastin, and mitomycin, or mixtures thereof.

The following examples are given to better illustrate the present invention.

**Example 1**

**3-[1-methyl-4[1-methyl-4[1-methyl-4[4-(2-chloroethylsulfinyl)phenyl-1-carboxamido]pyrrole-2-carboxamido]pyrrole-2-carboxamido]pyrrole-2-carboxamido]propionamidine**

***Step I:*** **The intermediate 4-(2-hydroxyethyl)thiobenzoic acid**

To a solution of 400 mg of 4-thiobenzoic acid in 2.85 ml of NaOH · 2N, 0.160 ml of 2-chloroethanol were added. The solution was refluxed for 1 hour, 2.85 ml of hydrochloric acid 2N were then added dropwise and the precipitated was filtered and dried yielding 370 mg of a white solid.

FAB-MS: m/z 220, (60, $(M+H]^+$)
PMR ($CDCl_3$) d:
7.61 (d, J= 15.7 Hz, 1H), 7.33 (m, 2H), 6.55 (m, 2H), 6.21 (d, J= 15.7 Hz, 1H), 4.22 (q, J=7.1 Hz, 2H), 3.9 (b.s., 1H), 3.19 (q, J=7.1 Hz, 2H), 1.25 (t, J=7.1 Hz, 3H), 1.28 (t, J=7.1 Hz, 3H).

By analogous procedures and by using the opportune starting materials the following intermediate compounds can be obtained:

3-methyl-4(2-hydroxyethyl)thiobenzoic acid;
4-(2-hydroxyethyl)thiocinnamic acid
FAB-MS: m/z 224
PMR (DMSO-$d_6$) d:
7.59 (m, 2H), 7.52 (d, J = 16.0 Hz; 1H), 7.31 (m, 2H), 6.46 (d, J= 16.0 Hz, 1H), 4.9 (bs, 1H), 3.57 (t, J=6.8 Hz, 2H), 3.08 (t, J=6.8 Hz, 2H).

***Step II:*** **The intermediate 4-(2-chloroethyl)thiobenzoic acid**

A solution of 400 mg of the intermediate, as prepared in step I, and 1.18 ml of thionyl chloride in 15 ml of toluene were refluxed for four hours, then the solvent was evaporated in vacuo. The crude residue was dissolved in 20 ml acetonitrile/water (1/1) and warmed at 40°C for 1 hour.

The solvent was then evaporated to dryness yielding 430 mg of a white solid which was used without further purification.

FAB-MS: m/z 216
PMR ($CDCl_3$) d:
8.01 (m, 2H) ; 7.38 (m, 2H); 3.67 (d, J = 7.0 Hz, 2H); 3.35 (d, J = 7.0 Hz, 2H).

By analogous procedures and by using the opportune starting materials the following compound can be obtained:

3-methyl-4(2-chloroethyl)thiobenzoic acid;

***Step III*****: The intermediate 4-(2-chloroethyl)sulfinylbenzoic acid**

A solution of 430 mg of the inermediate obtained from step II was added drpowise to a solution of 468 mg of $NaIO_4$ in 4.3 ml of water. The mixture was stirred at room temperature for 1 day, then at 80°C for 5 hours and subsequently dried under vacuum and purified by flash chromatogrphy (Ethylacetate/Exane:8/2) to yield 320 mg of the intermediate as a white solid.

By analogous procedures and by using the opportune starting materials the following compounds can be obtained:

3-methyl-4(2-chloroethyl)sulfinylbenzoic acid;
3-methyl-4(2-bromoethyl)sulfinylbenzoic acid;
4-(2-chloroethyl)sulfinylcinnamic acid.

***Step IV*****: The title compound**

86 mg of DCC were added to a solution of 106 mg of the intermediate obtained from step III in 4 ml of DMF and cooled at 0°C. The solution was stirred at 0°C for 30 minutes then 200 mg of 3-[1-methyl-4-[1-methyl-4-[1-methyl-4-aminopyrrole-2-carboxamido]pyrrole-2-carboxamido]pyrrole-2-carboxamidolpropionamidine dihydrochloride (prepared as reported in J. Med. Chem 32, 774-778, 1989) and 45 mg of potassium bicarbonate were added. The solution was stirred at room temperature for 3 hours then hydrochloric acid 2N was added up to pH acid.

The solvent was then removed in vacuo and the crude residue purified by flash chromatography (methylene chloride/methanol=85/15) to yield 150 mg of the title compound as a white solid.

FAB-MS: m/z 668, (100, $[M+H]^+$)
PMR (DMSO-$d_6$) d:
10.56 (s, 1H), 10.00 (s, 1H), 9.92 (s, 1H), 9.0 (b.s., 2H), 8.6 (b.s., 2H), 8.21 (t, J=5.6 Hz, 1H), 8.14 (m, 2H), 7.83 (m, 2H), 7.35 (d, J=1.7 Hz, 1H), 7.24 (d, J=1.7 Hz, 1H), 7.18 (d, J=1.7 Hz, 1H), 7.12 (d, J=1.7 Hz, 1H), 7.06 (d, J=1.7 Hz, 1H), 6.94 (d, J=1.7 Hz, 1H), 4.0-3.8 (m, 2H), 3.87 (s, 3H), 3.84 (s, 3H), 3.80 (s, 3H), 3.49 (m, 2H), 3.46 (m, 2H), 3.26 (m, 2H), 2.61 (t, J=6.6 Hz, 2H).

By analogous procedures and by using the opportune starting materials the following compounds can be obtained:

3-[1-methyl-4[1-methyl-4[1-methyl-4[4-(2-chloroethylsulfinyl)phenyl-1-carboxamido]pyrrole-2-carboxamido]pyrrole-2-carboxamido]pyrrole-2-carboxamido]propion-N-methylamidine;
3-[1-methyl-4[1-methyl-4[1-methyl-4[4-(2-chloroethylsulfinyl)phenyl-1-carboxamido]pyrrole-2-carboxamido]pyrrole-2-carboxamido]pyrrole-2-carboxamido]propion-N,N'-dimethylamidine;
3-[1-methyl-4[1-methyl-4[1-methyl-4[4-(2-chloroethylsulfinyl)phenyl-1-carboxamido]pyrrole-2-carboxamido]pyrrole-2-carboxamido]pyrrole-2-carboxamido]propion-N,N',N'-trimethylamidine;
3-[1-methyl-4[1-methyl-4[1-methyl-4[4-(2-chloroethylsulfinyl)phenyl-1-carboxamido]pyrrole-2-carboxamido]pyrrole-2-carboxamido]pyrrole-2-carboxamido]propion-N-cyanamidine;

3-[1-methyl-4[1-methyl-4[1-methyl-4[4-(2-chloroethylsulfinyl)phenyl-1-carboxamido]pyrrole-2-carboxamido]pyrrole-2-carboxamido]pyrrole-2-carboxamido]propionamidoxime;
3-[1-methyl-4[1-methyl-4[1-methyl-4[4-(2-chloroechylsulfinyl)phenyl-1-carboxamido]pyrrole-2-carboxamido]pyrrole-2-carboxamido]pyrrole-2-carboxamido]propionamide;
3-[1-methyl-4[1-methyl-4[1-methyl-4[4-(2-chloroethylsulfinyl)phenyl-1-carboxamido]pyrrole-2-carboxamido]pyrrole-2-carboxamido]pyrrole-2-carboxamido]propion-N-methylamide;
3-[1-methyl-4[1-methyl-4[1-methyl-4[4-(2-chloroethylsulfinyl)phenyl-1-carboxamido]pyrrole-2-carboxamido]pyrrole-2-carboxamido]pyrrole-2-carboxamido]propionitrile;
2-[1-methyl-4[1-methyl-4[1-methyl-4[4-(2-chloroethylsulfinyl)phenyl-1-carboxamido]pyrrole-2-carboxamido]pyrrole-2-carboxamido]pyrrole-2-carboxamido]ethylguanidine;
3-[1-methyl-4[1-methyl-4[1-methyl-4[4-(2-chloroethylsulfinyl)phenyl-1-carboxamido]pyrrole-2-carboxamido]pyrrole-2-carboxamido]pyrrole-2-carboxamido]propion-N,N-dimethylamine;
3-[1-methyl-4[1-methyl-4[1-methyl-4[4-(2-bromoethylsulfinyl)phenyl-1-carboxamido]pyrrole-2-carboxamido]pyrrole-2-carboxamido]pyrrole-2-carboxamido]propionamidine;
3-[1-methyl-4[1-methyl-4[1-methyl-4[3-methyl-4-(2-chloroethylsulfinyl)phenyl-1-carboxamido]pyrrole-2-carboxamido]pyrrole-2-carboxamido]pyrrole-2-carboxamido]propionamidine;
3-[1-methyl-4[1-methyl-4[1-methyl-4[4-(2-chloroethylsulfonyl)phenyl-1-carboxamido]pyrrole-2-carboxamido]pyrrole-2-carboxamido]pyrrole-2-carboxamido]propionamidine;
3-[1-methyl-4[1-methyl-4[1-methyl-4[4-(2-chloroethylsulfonyl)phenyl-1-carboxamido]pyrrole-2-carboxamido]pyrrole-2-carboxamido]pyrrole-2-carboxamido]propion-N-methylamidine;
3-[1-methyl-4[1-methyl-4[1-methyl-4[4-(2-chloroethylsulfonyl)phenyl-1-carboxamido]pyrrole-2-carboxamido]pyrrole-2-carboxamido]pyrrole-2-carboxamido]propion-N,N'-dimethylamidine;
3-[1-methyl-4[1-methyl-4[1-methyl-4[4-(2-chloroethylsulfonyl)phenyl-1-carboxamido]pyrrole-2-carboxamido]pyrrole-2-carboxamido]pyrrole-2-carboxamido]propion-N-cyanamidine;
3-[1-methyl-4[1-methyl-4[1-methyl-4[4-(2-chloroethylsulfonyl)phenyl-1-carboxamido]pyrrole-2-carboxamido]pyrrole-2-carboxamido]pyrrole-2-carboxamido]propionamidoxime;
3-[1-methyl-4[1-methyl-4[1-methyl-4[4-(2-chloroethylsulfonyl)phenyl-1-carboxamido]pyrrole-2-carboxamido]pyrrole-2-carboxamido]pyrrole-2-carboxamido]propionamide;
3-[1-methyl-4[1-methyl-4[1-methyl-4[4-(2-chloroethylsulfonyl)phenyl-1-carboxamido]pyrrole-2-carboxamido]pyrrole-2-carboxamido]pyrrole-2-carboxamido]propionitrile;
2-[1-methyl-4[1-methyl-4[1-methyl-4[4-(2-chloroethylsulfonyl)phenyl-1-carboxamido]pyrrole-2-carboxamido]pyrrole-2-carboxamido]pyrrole-2-carboxamido]ethylguanidine.

## Example 2

**3-[1-methyl-4[1-methyl-4[1-methyl-4[4-(2-chloroethylsulfinyl)cinnamoyl-1-carboxamido]pyrrole-2-carboxamido]pyrrole-2-carboxamido]pyrrolo-2-carboxamido]propionamidine**

***Step I*: The intermediate 4-(2-chloroethyl)thiocinnamic acid**

To a solution of 150 mg of 4-(2-hydroxyethyl)thiocinnamic acid (prepared as reported in example 1 step I) in 3 ml of pyridine, 0.105 mi of mesyl chloride were added and the solution was warmed for 2 hours at 80°C. The solution was cooled at room temperature and hydrochloric acid 37% was slowly added up to pH=1. The obtained precipitate was filtered and washed with water, then dried thus obtaining 100 mg of an orange solid.

FAB-MS: m/z 242
PMR (DMSO-$d_6$) d:
12.3 (bs, 1H); 7.63 (m, 2H); 7.54 (d, J = 15.9 Hz, 1H); 7.34 (m, 2H); 6.48 (d, J = 15.9 Hz, 1H); 3.76 (t, J = 7.1 Hz, 2H); 3.40 (t, J = 7.1 Hz, 2H).

By analogous procedures and by using the opportune starting materials the following products can be obtained:

4-(2-chloroethyl)thiobenzoic acid;
4-(2-bromoethyl)thiobenzoic acid;
3-methyl-4-(2-chloroethyl)thiobenzoic acid.

***Step II*: The intermediate 4-(2-chloroethyl)sulfinylcinnamic acid**

To 88 mg of $NaIO_4$ in 0.8 ml of water 90 mg of the intemediate obtained from step I, in 8 ml of MeOH, were added. The solution was warmed at 80°C for 5 hours then the solvent evaporated in vacuo. The residue was chromatographed on silica gel (Ethyl acetate/Exane:7/3) yielding 45 mg of a white solid.

***Step II*: The title compound**

A solution of 45 mg of 4-(2-chloroethyl)sulfinylcinnamic acid (prepared as described in step II), 35 mg of dicyclohexylcarbodiimide and 24 mg of 1-hydroxybenzotriazole hydrate in 3 ml of DMF, was stirred at 80°C for four hours, cooled at room temperature and then added with 90 mg 3-[1-methyl-4-[1-methyl-4-[1-methyl-4-aminopyrrole-2-carboxamido]pyrrole-2-carboxamido]pyrrole-2-carboxamido]propionamidine dihydrochloride (prepared as reported in J. Med. Chem 32, 774-778, 1989) and 17 mg of potassium bicarbonate.

The mixture was stirred at room temperature for 2 hours, the solvent was evaporated in vacuum and the crude residue purified by flash chromatography (methylene chloride/methanol: 8/2) to yield 100 mg of the title compound as a yellow solid.

FAB-MS: m/z 694, (100, $[M+H]^+$)
PMR ($DMSO-d_6$) d:
10.38 (s, 1H), 9.98 (s, 1H), 9.92 (s, 1H), 8.8 (b.s., 4H), 8.22 (t, J=6.0 Hz, 1H), 7.80 (m, 2H), 7.74 (m, 2H), 7.55 (d, J=15.6 Hz, 1H), 7.32 (d, J=1.7 Hz, 1H), 7.24 (d, J=1.7 Hz, 1H), 7.18 (d, J=1.7 Hz, 1H), 7.06 (d, J=1.7 Hz, 1H), 6.98 (d, J=1.7 Hz, 1H), 6.94 (d, J=1.7 Hz, 1H), 6.93 (d, J=15.6 Hz, 1H), 3.86 (s, 3H), 3.83 (s, 3H), 3.80 (s, 3H), 3.76-3.96 (m, 2H), 3.48 (m, 2H) , 3.2-3.45 (m, 2H), 2.61 (t, J=6.5 Hz, 2H).

By analogous procedures and by using the opportune starting materials the following products can be obtained:

3-[1-methyl-4[1-methyl-4[1-methyl-4[4-(2-chloroethylsulfinyl)cinnamoyl-1-carboxamido]pyrrole-2-carboxamido] pyrrole-2-carboxamido]pyrrole-2-carboxamido]propion-N-methylamidine;
3-[1-methyl-4[1-methyl-4[1-methyl-4[4-(2-chloroethylsulfinyl)cinnamoyl-1-carboxamido]pyrrole-2-carboxamido] pyrrole-2-carboxamido]pyrrole-2-carboxamido]propion-N,N'-dimethylamidine;
3-[1-methyl-4[1-methyl-4[1-methyl-4[4-(2-chloroethylsulfinyl)cinnamoyl-1-carboxamido]pyrrole-2-carboxamido] pyrrole-2-carboxamido]pyrrole-2-carboxamido]propion-N,N,-dimethylamidine;
3-[1-methyl-4[1-methyl-4[1-methyl-4[4-(2-chloroethylsulfinyl)cinnamoyl-1-carboxamido]pyrrole-2-carboxamido] pyrrole-2-carboxamido]pyrrole-2-carboxamido]propion-N-cyanamidine;
3-[1-methyl-4[1-methyl-4[1-methyl-4[4-(2-chloroethylsulfinyl)cinnamoyl-1-carboxamido]pyrrole-2-carboxamido] pyrrole-2-carboxamido]pyrrole-2-carboxamido]propionamidoxime;
3-[1-methyl-4[1-methyl-4[1-methyl-4[4-(2-chloroethylsulfinyl)cinnamoyl-1-carboxamido]pyrrole-2-carboxamido] pyrrole-2-carboxamido]pyrrole-2-carboxamido]propionamide;
3-[1-methyl-4[1-methyl-4[1-methyl-4[4-(2-chloroethylsulfinyl)cinnamoyl-1-carboxamido]pyrrole-2-carboxamido] pyrrole-2-carboxamido]pyrrole-2-carboxamido]propionamide;
3-[1-methyl-4[1-methyl-4[1-methyl-4[4-(2-chloroethylsulfinyl)cinnamoyl-1-carboxamido]pyrrole-2-carboxamido] pyrrole-2-carboxamido]pyrrole-2-carboxamido]propionitrile;
2-[1-methyl-4[1-methyl-4[1-methyl-4[4-(2-chloroethylsulfinyl)cinnamoyl-1-carboxamido]pyrrole-2-carboxamido] pyrrole-2-carboxamido]pyrrole-2-carboxamido]ethylguanidine;
3-[1-methyl-4[1-methyl-4[1-methyl-4[4-(2-chloroethylsulfinyl)cinnamoyl-1-carboxamido]pyrrole-2-carboxamido] pyrrole-2-carboxamido]pyrrole-2-carboxamido]propion-N,N,N'-trimethylamidine;
3-[1-methyl-4[1-methyl-4[1-methyl-4[4-(2-chloroethylsulfonyl)cinnamoyl-1-carboxamido]pyrrole-2-carboxamido] pyrrole-2-carboxamido]pyrrole-2-carboxamido]propionamidine;
3-[1-methyl-4[1-methyl-4[1-methyl-4[4-(2-chloroethylsulfonyl)cinnamoyl-1-carboxamido]pyrrole-2-carboxamido] pyrrole-2-carboxamido]pyrrole-2-carboxamido]propion-N-methylamidine;
3-[1-methyl-4[1-methyl-4[1-methyl-4[4-(2-chloroethylsulfonyl)cinnamoyl-1-carboxamido]pyrrole-2-carboxamido] pyrrole-2-carboxamido]pyrrole-2-carboxamido]propion-N,N'-dimethylamidine;
3-[1-methyl-4[1-methyl-4[1-methyl-4[4-(2-chloroethylsulfonyl)cinnamoyl-1-carboxamido]pyrrole-2-carboxamido] pyrrole-2-carboxamido]pyrrole-2-carboxamido]propion-N-cyanamidine;
3-[1-methyl-4[1-methyl-4[1-methyl-4[4-(2-chloroethylsulfonyl)cinnamoyl-1-carboxamido]pyrrole-2-carboxamido] pyrrole-2-carboxamido]pyrrole-2-carboxamido]propionamidoxime;
3-[1-methyl-4[1-methyl-4[1-methyl-4[4-(2-chloroethylsulfonyl)cinnamoyl-1-carboxamido]pyrrole-2-carboxamido]

pyrrole-2-carboxamido]pyrrole-2-carboxamido]propionamide;
3-[1-methyl-4[1-methyl-4[1-methyl-4[4-(2-chloroethylsulfonyl)cinnamoyl-1-carboxamido]pyrrole-2-carboxamido]pyrrole-2-carboxamido]pyrrole-2-carboxamido]propionitrile;
2-[1-methyl-4[1-methyl-4[1-methyl-4[4-(2-chloroethylsulfonyl)cinnamoyl-1-carboxamido]pyrrole-2-carboxamido]pyrrole-2-carboxamido]pyrrole-2-carboxamido]ethylguanidine.

**Example 3**

Tablets each weighing 0.250 g and containing 50 mg of the active substance can be manufactured as follows:

| *Composition for 10,000 tablets* | |
|---|---|
| 3-[1-methyl-4[1-methyl-4[1-methyl-4[4-(2-chloroethylsulfinyl)phenyl-1-carboxamido]pyrrole-2-carboxamido]pyrrole-2-carboxamido]pyrrole-2-carboxamido]propionamidine hydrochloride Lactose | 500 g<br>1.400 g |
| Corn starch | 500 g |
| Talc powder | 80 g |
| Magnesium stearate | 20 g |

The active substance, lactose and half of the corn starch were mixed; the mixture was then forced through a sieve of 0.5 mm mesh size.

Corn starch (10 g) was suspended in warm water (90 ml) and the resulting paste was used to granulate the powder. The granulate was dried, comminuted on a sieve of 1.4 mm mesh size, then the remaining quantity of starch, talc and magnesium stearate was added, carefully mixed and processed into tablets.

**Example 4**

Capsules, each dosed at 0.200 g and containing 20 mg of the active substance can be prepared as follows:

| *Composition for 500 capsules* | |
|---|---|
| 3-[1-methyl-4[1-methyl-4[1-methyl-4[4-(2-chloroethylsulfinyl)phenyl-1-carboxamido]pyrrole-2-carboxamido]pyrrole-2-carboxamido]pyrrole-2-carboxamido]propionamidine hydrochloride Lactose | 10 g<br>80 g |
| Corn starch | 5 g |
| Magnesium stearate | 5 g |

This formulation can be encapsulated in two-piece hard gelatin capsules and dosed at 0.200 g for each capsule.

**Example 5**

Intramuscular Injection 25 mg/ml

An injectable pharmaceutical composition can be manufactured by dissolving 25 g of 3-[1-methyl-4[1-methyl-4[1-methyl-4[4-(2-chloroethylsulfinyl)phenyl-1-carboxamido]pyrrole-2-carboxamido]pyrrole-2-carboxamido]pyrrole-2-carboxamido]propionamidine hydrochloride in sterile propyleneglycol (1000 ml) and sealing ampoules of 1-5 ml.

## Claims

1. A compound which is an oxidised sulfurated distamycin derivative of formula:

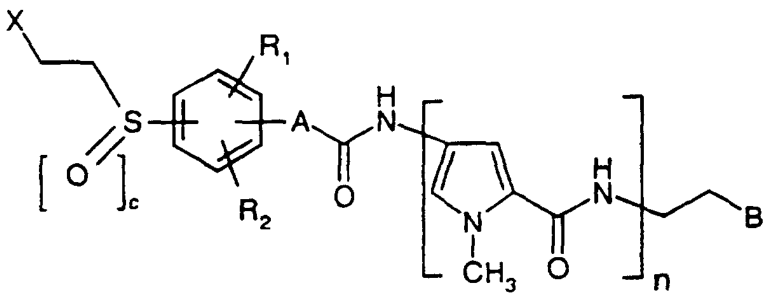

(I)

wherein:

n is 2, 3 or 4;
c is 1 or 2;
A is a bond, a $C_1$-$C_4$ alkylene or $C_2$-$C_4$ alkenylene group;
$R_1$ and $R_2$, which are the same or different, are selected from hydrogen, $C_1$-$C_4$ alkyl optionally substituted by one or more fluorine atoms, and $C_1$-$C_4$ alkoxy;
X is a halogen atom;
B is selected from:

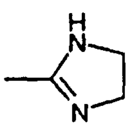 , 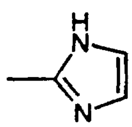 , 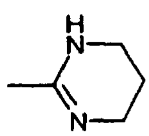 , 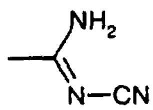 , 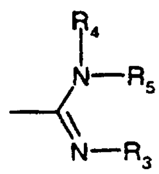 , 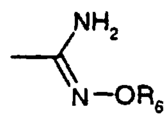 ,

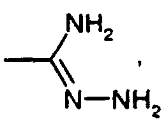 , —CN , 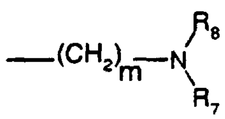 , 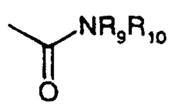 and 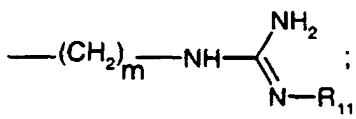 ;

wherein $R_3$, $R_4$, $R_5$, $R_6$, $R_7$, $R_8$, $R_9$ and $R_{10}$, which are the same or different, are selected from hydrogen or $C_1$-$C_4$ alkyl; $R_{11}$ is hydrogen, $C_1$-$C_4$ alkyl or hydroxy, and m is 0, 1 or 2; or pharmaceutically acceptable salts thereof.

**2.** A compound according to claim 1 wherein $R_1$, $R_4$, $R_5$, $R_6$, $R_7$, $R_2$, $R_9$, $R_{10}$ and $R_{11}$ are, independently from each other, hydrogen, methyl or ethyl.

**3.** A compound according to claim 1 or 2 wherein

n is 3;
c is 1;
A is a bond or vinylene;
$R_1$ and $R_2$, which are the same or different, are selected from hydrogen, methyl, methoxy or trifluoromethyl;
X is chloro or bromo;
B is selected from:

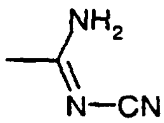 , 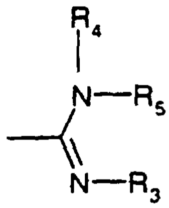 , 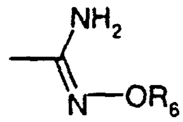 , 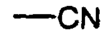 ,

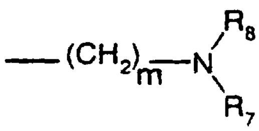 , 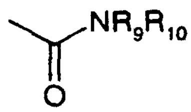 and 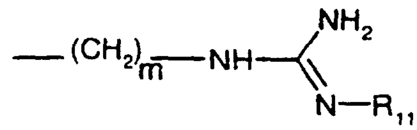

wherein $R_3$, $R_4$, $R_5$, $R_7$, $R_8$, $R_9$, $R_{10}$ and $R_{11}$, which are the same or different, are selected from hydrogen or methyl; $R_6$ is hydrogen; and m is 0 or 1;
or the pharmaceutically acceptable salts thereof.

**4.** A compound selected from the group consisting of:

3-[1-methyl-4[1-methyl-4[1-methyl-4[4-(2-chloroethylsulfinyl)phenyl-1-carboxamido]pyrrole-2-carboxamido]pyrrole-2-carboxamido]pyrrole-2-carboxamido]propionamidine;
3-[1-methyl-4[1-methyl-4[1-methyl-4[4-(2-chloroethylsulfinyl)phenyl-1-carboxamido]pyrrole-2-carboxamido]pyrrole-2-carboxamido]pyrrole-2-carboxamido]propion-N-methylamidine;
3-[1-methyl-4[1-methyl-4[1-methyl-4[4-(2-chloroethylsulfinyl)phenyl-1-carboxamido]pyrrole-2-carboxamido]pyrrole-2-carboxamido]pyrrole-2-carboxamido]propion-N,N'-dimethylamidine;
3-[1-methyl-4[1-methyl-4[1-methyl-4[4-(2-chloroethylsulfinyl)phenyl-1-carboxamido]pyrrole-2-carboxamido]pyrrole-2-carboxamido]pyrrole-2-carboxamido]propion-N,N',N'-trimethylamidine;
3-[1-methyl-4[1-methyl-4[1-methyl-4[4-(2-chloroethylsulfinyl)phenyl-1-carboxamido]pyrrole-2-carboxamido]pyrrole-2-carboxamido]pyrrole-2-carboxamido]propion-N-cyanamidine;
3-[1-methyl-4[1-methyl-4[1-methyl-4[4-(2-chloroethylsulfinyl)phenyl-1-carboxamido]pyrrole-2-carboxamido]pyrrole-2-carboxamido]pyrrole-2-carboxamido]propionamidoxime;
3-[1-methyl-4[1-methyl-4[1-methyl-4[4-(2-chloroethylsulfinyl)phenyl-1-carboxamido]pyrrole-2-carboxamido]pyrrole-2-carboxamido]pyrrole-2-carboxamido]propionamide;
3-[1-methyl-4[1-methyl-4[1-methyl-4[4-(2-chloroethylsulfinyl)phenyl-1-carboxamido]pyrrole-2-carboxamido]pyrrole-2-carboxamido]pyrrole-2-carboxamido]propion-N-methylamide;
3-[1-methyl-4[1-methyl-4[1-methyl-4[4-(2-chloroethylsulfinyl)phenyl-1-carboxamido]pyrrole-2-carboxamido]pyrrole-2-carboxamido]pyrrole-2-carboxamido]propionitrile;
2-[1-methyl-4[1-methyl-4[1-methyl-4[4-(2-chloroethylsulfinyl)phenyl-1-carboxamido]pyrrole-2-carboxamido]pyrrole-2-carboxamido]pyrrole-2-carboxamido]ethylguanidine;
3-[1-methyl-4[1-methyl-4[1-methyl-4[4-(2-chloroethylsulfinyl)phenyl-1-carboxamido]pyrrole-2-carboxamido]pyrrole-2-carboxamido]pyrrole-2-carboxamido]propion-N,N-dimethylamine;
3-[1-methyl-4[1-methyl-4[1-methyl-4[4-(2-bromoethylsulfinyl)phenyl-1-carboxamido]pyrrole-2-carboxamido]pyrrole-2-carboxamido]pyrrole-2-carboxamido]propionamidine;
3-[1-methyl-4[1-methyl-4[1-methyl-4[3-methyl-4-(2-chloroethylsulfinyl)phenyl-1-carboxamido]pyrrole-2-carboxamido]pyrrole-2-carboxamido]pyrrole-2-carboxamido]propionamidine;
3-[1-methyl-4[1-methyl-4[1-methyl-4[4-(2-chloroethylsulfinyl)cinnamoyl-1-carboxamido]pyrrole-2-carboxamido]pyrrole-2-carboxamido]pyrrole-2-carboxamido]propionamidine;
3-[1-methyl-4[1-methyl-4[1-methyl-4[4-(2-chloroethylsulfinyl)cinnamoyl-1-carboxamido]pyrrole-2-carboxamido]pyrrole-2-carboxamido]pyrrole-2-carboxamido]propion-N-methylamidine;
3-[1-methyl-4[1-methyl-4[1-methyl-4[4-(2-chloroethylsulfinyl)cinnamoyl-1-carboxamido]pyrrole-2-carboxamido]pyrrole-2-carboxamido]pyrrole-2-carboxamido]propion-N,N'-dimethylamidine;
3-[1-methyl-4[1-methyl-4[1-methyl-4[4-(2-chloroethylsulfinyl)cinnamoyl-1-carboxamido]pyrrole-2-carboxamido]pyrrole-2-carboxamido]pyrrole-2-carboxamido]propion-N,N,-dimethylamidine;
3-[1-methyl-4[1-methyl-4[1-methyl-4[4-(2-chloroethylsulfinyl)cinnamoyl-1-carboxamido]pyrrole-2-carboxamido]pyrrole-2-carboxamido]pyrrole-2-carboxamido]propion-N-cyanamidine;

3-[1-methyl-4[1-methyl-4[1-methyl-4[4-(2-chloroethylsulfinyl)cinnamoyl-1-carboxamido]pyrrole-2-carboxamido]pyrrole-2-carboxamido]pyrrole-2-carboxamido]propionamidoxime;
3-[1-methyl-4[1-methyl-4[1-methyl-4[4-(2-chloroethylsulfinyl)cinnamoyl-1-carboxamido]pyrrole-2-carboxamido]pyrrole-2-carboxamido]pyrrole-2-carboxamido]propionamide;
3-[1-methyl-4[1-methyl-4[1-methyl-4[4-(2-chloroethylsulfinyl)cinnamoyl-1-carboxamido]pyrrole-2-carboxamido]pyrrole-2-carboxamido]pyrrole-2-carboxamido]propionamide;
3-[1-methyl-4[1-methyl-4[1-methyl-4[4-(2-chloroethylsulfinyl)cinnamoyl-1-carboxamido]pyrrole-2-carboxamido]pyrrole-2-carboxamido]pyrrole-2-carboxamido]propionitrile;
2-[1-methyl-4[1-methyl-4[1-methyl-4[4-(2-chloroethylsulfinyl)cinnamoyl-1-carboxamido]pyrrole-2-carboxamido]pyrrole-2-carboxamido]pyrrole-2-carboxamido]ethylguanidine;
3-[1-methyl-4[1-methyl-4[1-methyl-4[4-(2-chloroethylsulfinyl)cinnamoyl-1-carboxamido]pyrrole-2-carboxamido)pyrrole-2-carboxamido]pyrrole-2-carboxamido]propion-N,N,N'-trimethylamidine;
3-[1-methyl-4[1-methyl-4[1-methyl-4[4-(2-chloroethylsulfonyl)phenyl-1-carboxamido]pyrrole-2-carboxamido]pyrrole-2-carboxamido]pyrrole-2-carboxamido]propionamidine;
3-[1-methyl-4[1-methyl-4[1-methyl-4[4-(2-chloroethylsulfonyl)phenyl-1-carboxamido]pyrrole-2-carboxamido]pyrrole-2-carboxamido]pyrrole-2-carboxamido]propion-N-methylamidine;
3-[1-methyl-4[1-methyl-4[1-methyl-4[4-(2-chloroethylsulfonyl)phenyl-1-carboxamido]pyrrole-2-carboxamido]pyrrole-2-carboxamido]pyrrole-2-carboxamido]propion-N,N'-dimethylamidine;
3-[1-methyl-4[1-methyl-4[1-methyl-4[4-(2-chloroethylsulfonyl)phenyl-1-carboxamido]pyrrole-2-carboxamido]pyrrole-2-carboxamido]pyrrole-2-carboxamido]propion-N-cyanamidine;
3-[1-methyl-4[1-methyl-4[1-methyl-4[4-(2-chloroethylsulfonyl)phenyl-1-carboxamido]pyrrole-2-carboxamido]pyrrole-2-carboxamido]pyrrole-2-carboxamido]propionamidoxime;
3-[1-methyl-4[1-methyl-4[1-methyl-4[4-(2-chloroethylsulfonyl)phenyl-1-carboxamido]pyrrole-2-carboxamido]pyrrole-2-carboxamido]pyrrole-2-carboxamido]propionamide;
3-[1-methyl-4[1-methyl-4[1-methyl-4[4-(2-chloroethylsulfonyl)phenyl-1-carboxamido]pyrrole-2-carboxamido)pyrrole-2-carboxamido]pyrrole-2-carboxamido]propionitrile;
2-[1-methyl-4[1-methyl-4[1-methyl-4[4-(2-chloroethylsulfonyl)phenyl-1-carboxamido]pyrrole-2-carboxamido]pyrrole-2-carboxamido]pyrrole-2-carboxamido]ethylguanidine;
3-[1-methyl-4[1-methyl-4[1-methyl-4[4-(2-chloroethylsulfonyl)cinnamoyl-1-carboxamido]pyrrole-2-carboxamido]pyrrole-2-carboxamido]pyrrole-2-carboxamido]propionamidine;
3-[1-methyl-4[1-methyl-4[1-methyl-4[4-(2-chloroethylsulfonyl)cinnamoyl-1-carboxamido]pyrrole-2-carboxamido]pyrrole-2-carboxamido]pyrrole-2-carboxamido]propion-N-methylamidine;
3-[1-methyl-4[1-methyl-4[1-methyl-4[4-(2-chloroethylsulfonyl)cinnamoyl-1-carboxamido]pyrrole-2-carboxamido]pyrrole-2-carboxamido]pyrrole-2-carboxamido]propion-N,N'-dimethylamidine;
3-[1-methyl-4[1-methyl-4[1-methyl-4[4-(2-chloroethylsulfonyl)cinnamoyl-1-carboxamido]pyrrole-2-carboxamido]pyrrole-2-carboxamido]pyrrole-2-carboxamido]propion-N-cyanamidine;
3-[1-methyl-4[1-methyl-4[1-methyl-4[4-(2-chloroethylsulfonyl)cinnamoyl-1-carboxamido]pyrrole-2-carboxamido]pyrrole-2-carboxamido]pyrrole-2-carboxamido]propionamidoxime;
3-[1-methyl-4[1-methyl-4[1-methyl-4[4-(2-chloroethylsulfonyl)cinnamoyl-1-carboxamido]pyrrole-2-carboxamido]pyrrole-2-carboxamido]pyrrole-2-carboxamido]propionamide;
3-[1-methyl-4[1-methyl-4[1-methyl-4[4-(2-chloroethylsulfonyl)cinnamoyl-1-carboxamido]pyrrole-2-carboxamido]pyrrole-2-carboxamido]pyrrole-2-carboxamido]propionitrile;
2-[1-methyl-4[1-methyl-4[1-methyl-4[4-(2-chloroethylsulfonyl)cinnamoyl-1-carboxamido]pyrrole-2-carboxamido]pyrrole-2-carboxamido]pyrrole-2-carboxamido]ethylguanidine; and the pharmaceutically acceptable salts thereof.

**5.** A process for preparing the compounds of formula (I), and the pharmaceutically acceptable salts thereof, which process comprises:

(a) when B is other than

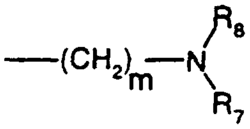

and

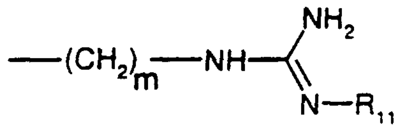

reaching a compound of formula:

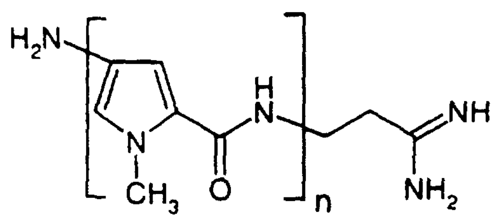

(II)

with a compound of formula:

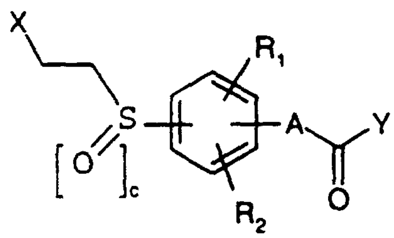

(III)

wherein n, c, $R_1$, $R_2$, X and A are as defined in claim 1, and Y is hydroxy or a suitable leaving group; so as to obtain a compound of formula:

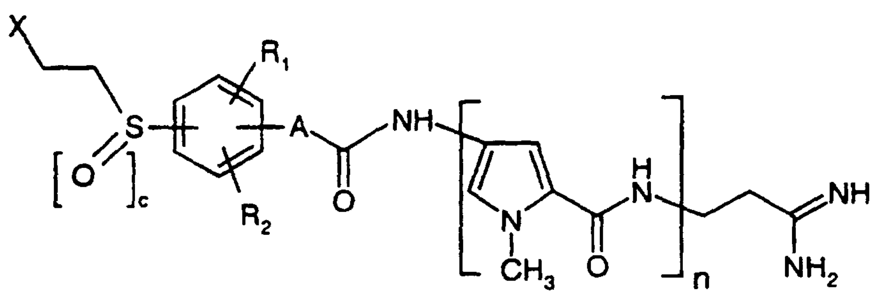

(Ia)

and, then, optionally reacting a compound of formula (Ia) with:

(i) $H_2N-(CH_2)_r-NH_2$, wherein r is 2 or 3, so as to obtain a compound of formula (I) having B equal to:

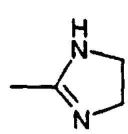

or

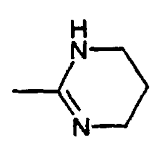

(ii) $H_2N-CH_2-CHO$, so obtaining a compound of formula (I) having B equal to:

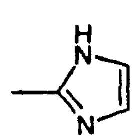

(iii)$H_2N-CN$, so obtaining a compound of formula (I) having B equal to:

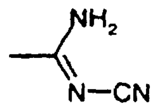

(iv) $H_2N\text{-}OR_6$, so obtaining a compound of formula (I) having B equal to:

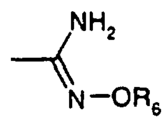

(v) $H_2N\text{-}NH_2$, so obtaining a compound of formula (I) having B equal to:

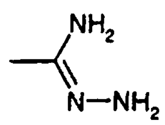

(vi) $HNR_4R_5$, so obtaining a compound of formula (I) having B equal to:

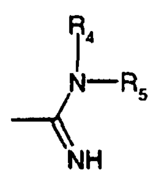

and then optionally with $H_2NR_3$, so obtaining a compound of formula (I) having B equal to:

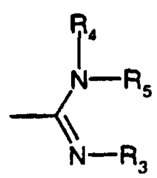

(vii)succinic anhydride, so obtaining a compound of formula (I) having B equal to -C≡N;
(viii)water in an alkaline medium, so obtaining a compound of formula (I) having B equal to $-CONR_9R_{10}$ wherein $R_9$ and $R_{10}$ are both hydrogen atoms;
(ix) $HNR_9R_{10}$, so obtaining a compound of formula (I) having B equal to:

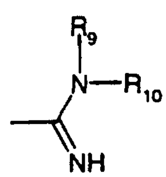

and then with water in an alkaline medium, so obtaining a compound of formula (I) having B equal

to - $CONR_9R_{10}$, wherein $R_9$ and $R_{10}$ are as defined in claim 1;
or

(b) when B is other than

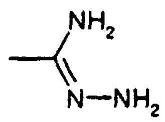

reacting a compound of formula:

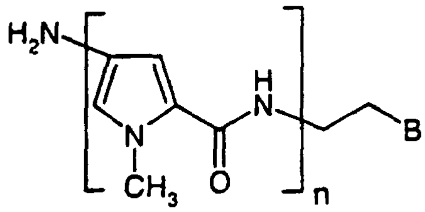

(IV)

with a compound of formula:

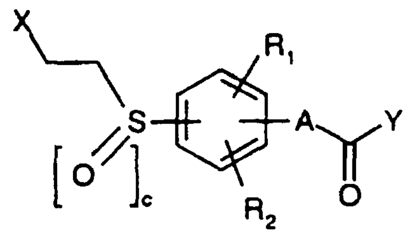

(III)

wherein n, c, B, $R_1$, $R_2$, X, Y and A are as defined above;
so obtaining the corresponding compound of formula (I);
and, if desired, converting the compound of formula (I) into a pharmaceutically acceptable salt thereof.

**6.** A process according to claim 5 wherein, in the compounds of formula (III), Y is hydroxy or a group selected from chloro, 2,4,5-trichlorophenoxy, 2,4-dinitro-phenoxy, succinimido-N-oxy and imidazolyl.

**7.** A pharmaceutical composition comprising one or more pharmaceutically acceptable carriers and/or diluents and, as the active principle, a compound as defined in claim 1.

**8.** A compound as defined in claim 1 for use in a method of treatment of the human or animal body by therapy.

**9.** A compound as defined in claim 8 for use as an antitumor agent.

**10.** Use of a compound as defined in claim 1 in the manufacture of a medicament for use as an antitumor agent.

**Patentansprüche**

**1.** Eine Verbindung, welche ein oxidiertes Schwefeldistamycinderivat der folgenden Formel ist:

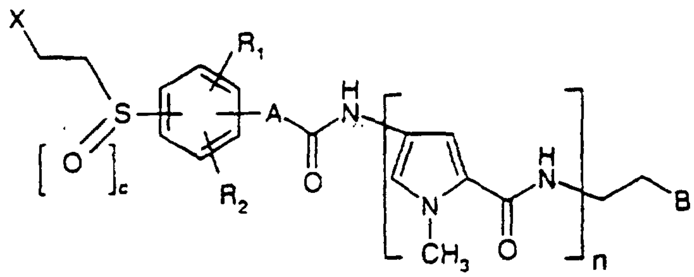

(I)

worin:

n 2, 3 oder 4 ist;
c ist 1 oder 2;
A ist eine Bindung, eine $C_1$-$C_4$ Alkylen- oder $C_2$-$C_4$ Alkenylengruppe;
$R_1$ und $R_2$, welche gleich oder unterschiedlich sind, sind gewählt aus Wasserstoff, $C_1$-$C_4$ Alkyl wahlweise substituiert mit einem oder mehreren Fluoratomen, und $C_1$-$C_4$ Alkoxy;
X ist ein Halogenatom;
B ist gewählt aus:

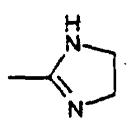 , 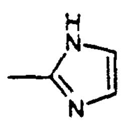 , 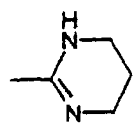 , 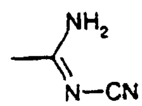 , 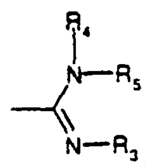 , 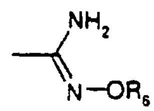 ,

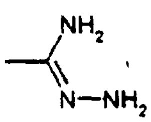 , —CN , 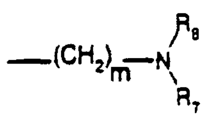 , 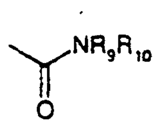 und 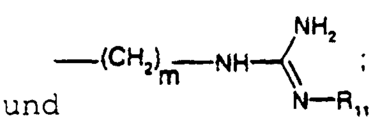 ;

worin $R_3$, $R_4$, $R_5$, $R_6$, $R_7$, $R_8$, $R_9$ und $R_{10}$, welche gleich oder unterschiedlich sind, gewählt sind aus Wasserstoff oder $C_1$-$C_4$ Alkyl; $R_{11}$ ist Wasserstoff, $C_1$-$C_4$ Alkyl oder Hydroxy, und m ist 0, 1 oder 2; oder pharmazeutisch verträgliche Salze davon.

**2.** Eine Verbindung gemäß Anspruch 1, worin $R_3$, $R_4$, $R_5$, $R_6$, $R_7$, $R_8$, $R_9$, $R_{10}$ und $R_{11}$ unabhängig voneinander Wasserstoff, Methyl oder Ethyl sind.

**3.** Eine Verbindung gemäß Anspruch 1 oder 2, worin n 3 ist;

c ist 1;
A ist eine Bindung oder Vinylen;
$R_1$ und $R_2$, welche gleich oder unterschiedlich sind, sind gewählt aus Wasserstoff, Methyl, Methoxy oder Trifluormethyl;
X ist Chlor oder Brom;
B ist gewählt aus:

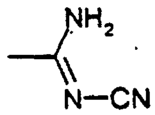,
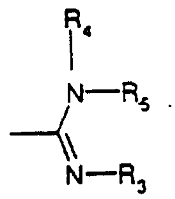,
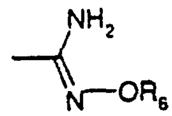,
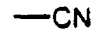,

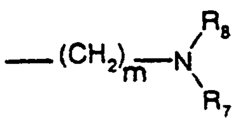,
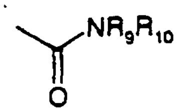
und
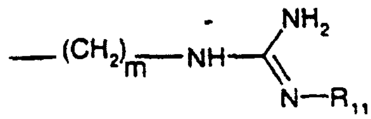

worin $R_3$, $R_4$, $R_5$, $R_7$, $R_8$, $R_9$, $R_{10}$ und $R_{11}$, welche gleich oder unterschiedlich sind, gewählt sind aus Wasserstoff oder Methyl; $R_6$ ist Wasserstoff; und m ist 0 oder 1; oder die pharmazeutisch verträglichen Salze davon.

**4.** Eine Verbindung gewählt aus der Gruppe bestehend aus:

3-[1-Methyl-4[1-methyl-4[1-methyl-4[4-(2-chlorethylsulfinyl)phenyl-1-carboxamido]pyrrol-2-carboxamido]pyrrol-2-carboxamido]pyrrol-2-carboxamido]propionamidin;
3-[1-Methyl-4[1-methyl-4[1-methyl-4[4-(2-chlorethylsulfinyl)phenyl-1-carboxamido]pyrrol-2-carboxamido]pyrrol-2-carboxamido]pyrrol-2-carboxamido]propion-N-methylamidin;
3-[1-Methyl-4[1-methyl-4[1-methyl-4[4-(2-chlorethylsulfinyl)phenyl-1-carboxamido]pyrrol-2-carboxamido]pyrrol-2-carboxamido]pyrrol-2-carboxamido]propion-N,N'-dimethylamidin;
3-[1-Methyl-4[1-methyl-4[1-methyl-4[4-(2-chlorethylsulfinyl)phenyl-1-carboxamido]pyrrol-2-carboxamido]pyrrol-2-carboxamido]pyrrol-2-carboxamido]propion-N,N',N'-trimethylamidin;
3-[1-Methyl-4[1-methyl-4[1-methyl-4[4-(2-chlorethylsulfinyl)phenyl-1-carboxamido]pyrrol-2-carboxamido]pyrrol-2-carboxamido]pyrrol-2-carboxamido]propion-N-cyanamidin;
3-[1-Methyl-4[1-methyl-4[1-methyl-4[4-(2-chlorethylsulfinyl)phenyl-1-carboxamido]pyrrol-2-carboxamido]pyrrol-2-carboxamido]pyrrol-2-carboxamido]propionamidoxim;
3-[1-Methyl-4[1-methyl-4[1-methyl-4[4-(2-chlorethylsulfinyl)phenyl-1-carboxamido]pyrrol-2-carboxamido]pyrrol-2-carboxamido]pyrrol-2-carboxamido]propionamid;
3-[1-Methyl-4[1-methyl-4[1-methyl-4[4-(2-chlorethylsulfinyl)phenyl-1-carboxamido]pyrrol-2-carboxamido]pyrrol-2-carboxamido]pyrrol-2-carboxamido]propion-N-methylamid;
3-[1-Methyl-4[1-methyl-4[1-methyl-4[4-(2-chlorethylsulfinyl)phenyl-1-carboxamido]pyrrol-2-carboxamido]pyrrol-2-carboxamido]pyrrol-2-carboxamido]propionitril;
2-[1-Methyl-4[1-methyl-4[1-methyl-4[4-(2-chlorethylsulfinyl)phenyl-1-carboxamido]pyrrol-2-carboxamido]pyrrol-2-carboxamido]pyrrol-2-carboxamido]ethylguanidin;
3-[1-Methyl-4[1-methyl-4[1-methyl-4[4-(2-chlorethylsulfinyl)phenyl-1-carboxamido]pyrrol-2-carboxamido]pyrrol-2-carboxamido]pyrrol-2-carboxamido]propion-N,N-dimethylamin;
3-[1-Methyl-4[1-methyl-4[1-methyl-4[4-(2-bromethylsulfinyl)phenyl-1-carboxamido]pyrrol-2-carboxamido]pyrrol-2-carboxamido]pyrrol-2-carboxamido]propionamidin;
3-[1-Methyl-4[1-methyl-4[1-methyl-4[3-methyl-4-(2-chlorethylsulfinyl)phenyl-1-carboxamido]pyrrol-2-carboxamido]pyrrol-2-carboxamido]pyrrol-2-carboxamido]propionamidin;
3-[1-Methyl-4[1-methyl-4[1-methyl-4[4-(2-chlorethylsulfinyl)cinnamoyl-1-carboxamido]pyrrol-2-carboxamido]pyrrol-2-carboxamido]pyrrol-2-carboxamido]propionamidin;
3-[1-Methyl-4[1-methyl-4[1-methyl-4[4-(2-chlorethylsulfinyl)cinnamoyl-1-carboxamido]pyrrol-2-carboxamido]pyrrol-2-carboxamido]pyrrol-2-carboxamido]propion-N-methylamidin;
3-[1-Methyl-4[1-methyl-4[1-methyl-4[4-(2-chlorethylsulfinyl)cinnamoyl-1-carboxamido]pyrrol-2-carboxamido]pyrrol-2-carboxamido]pyrrol-2-carboxamido]propion-N,N'-dimethylamidin;
3-[1-Methyl-4[1-methyl-4[1-methyl-4[4-(2-chlorethylsulfinyl)cinnamoyl-1-carboxamido]pyrrol-2-carboxamido]pyrrol-2-carboxamido]pyrrol-2-carboxamido]propion-N,N,-dimethylamidin;
3-[1-Methyl-4[1-methyl-4[1-methyl-4[4-(2-chlorethylsulfinyl)cinnamoyl-1-carboxamido]pyrrol-2-carboxamido]pyrrol-2-carboxamido]pyrrol-2-carboxamido]propion-N-cyanamidin;
3-[1-Methyl-4[1-methyl-4[1-methyl-4[4-(2-chlorethylsulfinyl)cinnamoyl-1-carboxamido]pyrrol-2-carboxamido]pyrrol-2-carboxamido]pyrrol-2-carboxamido]propionamidoxim;
3-[1-Methyl-4[1-methyl-4[1-methyl-4[4-(2-chlorethylsulfinyl)cinnamoyl-1-carboxamido]pyrrol-2-carboxamido]pyrrol-2-carboxamido]pyrrol-2-carboxamido]propionamid;
3-[1-Methyl-4[1-methyl-4[1-methyl-4[4-(2-chlorethylsulfinyl)cinnamoyl-1-carboxamido]pyrrol-2-carboxamido]pyrrol-2-carboxamido]pyrrol-2-carboxamido]propionamid;
3-[1-Methyl-4[1-methyl-4[1-methyl-4[4-(2-chlorethylsulfinyl)cinnamoyl-1-carboxamido]pyrrol-2-carboxamido]pyrrol-2-carboxamido]pyrrol-2-carboxamido]propionitril;
2-[1-Methyl-4[1-methyl-4[1-methyl-4[4-(2-chlorethylsulfinyl)cinnamoyl-1-carboxamido]pyrrol-2-carboxamido]pyrrol-2-carboxamido]pyrrol-2-carboxamido]ethylguanidin;
3-[1-Methyl-4[1-methyl-4[1-methyl-4[4-(2-chlorethylsulfinyl)cinnamoyl-1-carboxamido]pyrrol-2-carboxamido]pyrrol-2-carboxamido]pyrrol-2-carboxamido]propion-N,N,N'-trimethylamidin;
3-[1-Methyl-4[1-methyl-4[1-methyl-4[4-(2-chlorethylsulfonyl)phenyl-1-carboxamido]pyrrol-2-carboxamido]pyrrol-2-carboxamido]pyrrol-2-carboxamido]propionamidin;
3-[1-Methyl-4[1-methyl-4[1-methyl-4[4-(2-chlorethylsulfonyl)phenyl-1-carboxamido]pyrrol-2-carboxamido]pyrrol-2-carboxamido]pyrrol-2-carboxamido]propion-N-methylamidin;
3-[1-Methyl-4[1-methyl-4[1-methyl-4[4-(2-chlorethylsulfonyl)phenyl-1-carboxamido]pyrrol-2-carboxamido]pyrrol-2-carboxamido]pyrrol-2-carboxamido]propion-N,N'-dimethylamidin;
3-[1-Methyl-4[1-methyl-4[1-methyl-4[4-(2-chlorethylsulfonyl)phenyl-1-carboxamido]pyrrol-2-carboxamido]pyrrol-2-carboxamido]pyrrol-2-carboxamido]propion-N-cyanamidin;
3-[1-Methyl-4[1-methyl-4[1-methyl-4[4-(2-chlorethylsulfonyl)phenyl-1-carboxamido]pyrrol-2-carboxamido]pyrrol-2-carboxamido]pyrrol-2-carboxamido]propionamidoxim;
3-[1-Methyl-4[1-methyl-4[1-methyl-4[4-(2-chlorethylsulfonyl)phenyl-1-carboxamido]pyrrol-2-carboxamido]pyrrol-2-carboxamido]pyrrol-2-carboxamido]propionamid;
3-[1-Methyl-4[1-methyl-4[1-methyl-4[4-(2-chlorethylsulfonyl)phenyl-1-carboxamido]pyrrol-2-carboxamido]pyrrol-2-carboxamido]pyrrol-2-carboxamido]propionitril;
2-[1-Methyl-4[1-methyl-4[1-methyl-4[4-(2-chlorethylsulfonyl)phenyl-1-carboxamido]pyrrol-2-carboxamido]pyrrol-2-carboxamido]pyrrol-2-carboxamido]ethylguanidin;
3-[1-Methyl-4[1-methyl-4[1-methyl-4[4-(2-chlorethylsulfonyl)cinnamoyl-1-carboxamido]pyrrol-2-carboxamido]pyrrol-2-carboxamido]pyrrol-2-carboxamido]propionamidin;
3-[1-Methyl-4[1-methyl-4[1-methyl-4[4-(2-chlorethylsulfonyl)cinnamoyl-1-carboxamido]pyrrol-2-carboxamido]pyrrol-2-carboxamido]pyrrol-2-carboxamido]propion-N-methylamidin;
3-[1-Methyl-4[1-methyl-4[1-methyl-4[4-(2-chlorethylsulfonyl)cinnamoyl-1-carboxamido]pyrrol-2-carboxamido]pyrrol-2-carboxamido]pyrrol-2-carboxamido]propion-N,N'-dimethylamidin;
3-[1-Methyl-4[1-methyl-4[1-methyl-4[4-(2-chlorethylsulfonyl)cinnamoyl-1-carboxamido]pyrrol-2-carboxamido]pyrrol-2-carboxamido]pyrrol-2-carboxamido]propion-N-cyanamidin;
3-[1-Methyl-4[1-methyl-4[1-methyl-4[4-(2-chlorethylsulfonyl)cinnamoyl-1-carboxamido]pyrrol-2-carboxamido]pyrrol-2-carboxamido]pyrrol-2-carboxamido]propionamidoxim;
3-[1-Methyl-4[1-methyl-4[1-methyl-4[4-(2-chlorethylsulfonyl)cinnamoyl-1-carboxamido]pyrrol-2-carboxamido]pyrrol-2-carboxamido]pyrrol-2-carboxamido]propionamid;
3-[1-Methyl-4[1-methyl-4[1-methyl-4[4-(2-chlorethylsulfonyl)cinnamoyl-1-carboxamido]pyrrol-2-carboxamido]pyrrol-2-carboxamido]pyrrol-2-carboxamido]propionitril;
2-[1-Methyl-4[1-methyl-4[1-methyl-4[4-(2-chlorethylsulfonyl)cinnamoyl-1-carboxamido]pyrrol-2-carboxamido]pyrrol-2-carboxamido]pyrrol-2-carboxamido]ethylguanidin; und die pharmazeutisch verträglichen Salze davon.

**5.** Ein Verfahren zur Herstellung der Verbindungen von Formel (I) und der pharmazeutisch verträglichen Salze davon, wobei das Verfahren folgendes umfasst:

(a) wenn B nicht

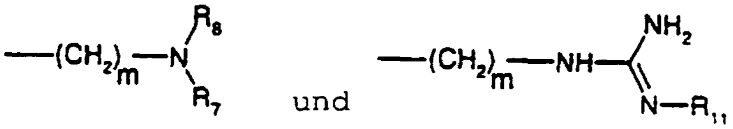

ist, Reagieren einer Verbindung mit der Formel:

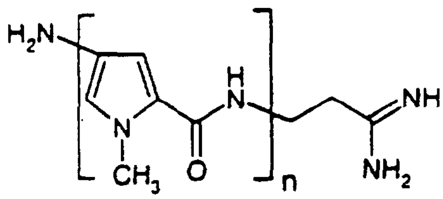

(II)

mit einer Verbindung der Formel:

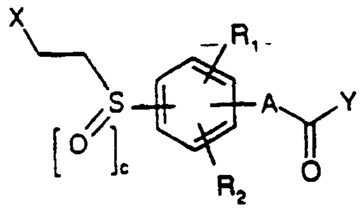

(III)

worin n, c, $R_1$, $R_2$, X und A wie in Anspruch 1 definiert sind, und Y ist Hydroxy oder eine geeignete Abgangsgruppe;
um eine Verbindung mit folgender Formel zu erhalten:

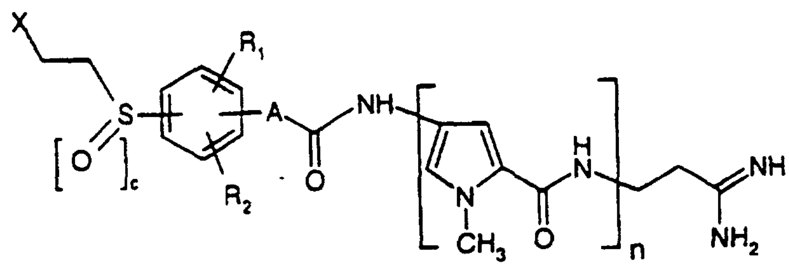

(Ia)

und dann wahlweise Reagieren einer Verbindung der Formel (Ia) mit:

(i) $H_2N-(CH_2)_r-NH_2$, worin r 2 oder 3 ist, um eine Verbindung der Formel (I) zu erhalten, in der B folgendes ist:

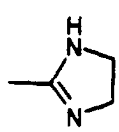

oder

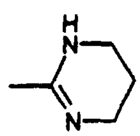

(ii) $H_2N-CH_2-CHO$, um eine Verbindung der Formel (I) zu erhalten, in der B folgendes ist:

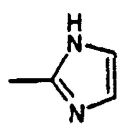

(iii) $H_2N-CN$, um eine Verbindung der Formel (I) zu erhalten, in der B folgendes ist:

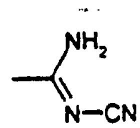

(iv) $H_2N\text{-}OR_6$, um eine Verbindung der Formel (I) zu erhalten, in der B folgendes ist:

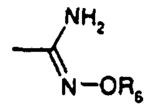

(v) $H_2N\text{-}NH_2$, um eine Verbindung der Formel (I) zu erhalten, in der B folgendes ist:

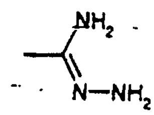

(vi) $HNR_4R_5$, um eine Verbindung der Formel (I) zu erhalten, in der B folgendes ist:

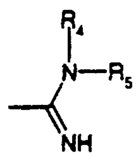

und dann wahlweise mit $H_2NR_3$, um eine Verbindung der Formel (I) zu erhalten, in der B folgendes ist:

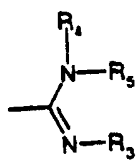

(vii) Bernsteinsäureanhydrid, um eine Verbindung der Formel (I) zu erhalten, in der B gleich -C≡N ist;
(viii) Wasser in einem alkalischen Medium, um eine Verbindung der Formel (I) zu erhalten, in der B gleich $-CONR_9R_{10}$ ist, worin $R_9$ und $R_{10}$ beide Wasserstoffatome sind;
(ix) $HNR_9R_{10}$, um eine Verbindung der Formel (I) zu erhalten, in der B folgendes ist:

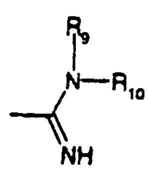

und dann mit Wasser in einem alkalischen Medium, um eine Verbindung der Formel (I) zu erhalten, in der B gleich $-CONR_9R_{10}$ ist, worin $R_9$ und $R_{10}$ wie in Anspruch 1 definiert sind; oder

(b) wenn B nicht

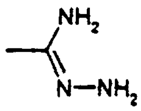

ist, Reagieren einer Verbindung der Formel:

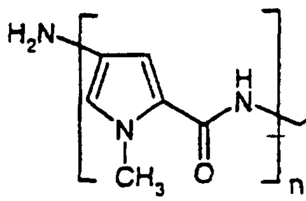

(IV)

mit einer Verbindung der Formel:

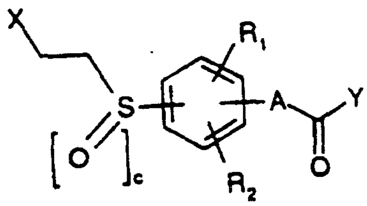

(III)

worin n, c, B, $R_1$, $R_2$, X, Y und A wie oben definiert sind;
um die entsprechende Verbindung der Formel (I) zu erhalten; und, wenn gewünscht, Umwandeln der Verbindung der Formel (I) in ein pharmazeutisch verträgliches Salz davon.

**6.** Ein Verfahren. gemäß Anspruch 5, worin, in den Verbindungen der Formel (III), Y Hydroxy ist oder eine Gruppe gewählt aus Chlor, 2,4,5-Trichlorphenoxy, 2,4-Dinitro-phenoxy, Succinimido-N-oxy und Imdiazolyl.

**7.** Eine pharmazeutische Zusammensetzung, die einen oder mehrere pharmazeutisch verträgliche Träger und/oder Verdünnungsmittel und, als aktiven Grundbestandteil, eine Verbindung wie in Anspruch 1 definiert, umfasst.

**8.** Eine Verbindung wie in Anspruch 1 definiert, für die Verwendung in einem Verfahren zur Behandlung des menschlichen oder tierischen Körpers durch Therapie.

**9.** Eine Verbindung wie in Anspruch 8 definiert, für die Verwendung als ein Anti-Tumormittel.

**10.** Verwendung einer Verbindung wie in Anspruch 1 definiert, in der Herstellung eines Medikaments für die Verwendung als ein Anti-Tumormittel.

**Revendications**

**1.** Composé qui est un dérivé de distamycine sulfuré oxydé de formule :

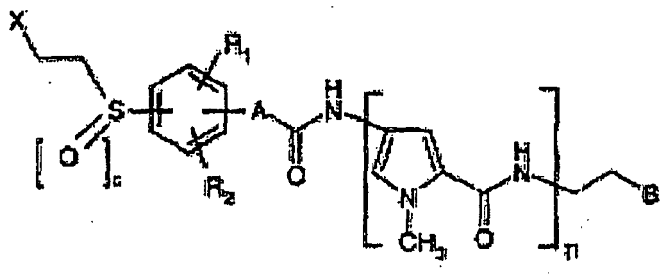 (I)

dans laquelle :

n est égal à 2, 3 ou 4 ;
c est égal à 1 ou 2 ;
A représente une liaison, un groupe alkylène en $C_1$ à $C_4$ ou alcénylène en $C_2$ à $C_4$ ;
$R_1$ et $R_2$, qui sont identiques ou différents, sont choisis entre l'hydrogène, un groupe alkyle en $C_1$ à $C_4$ facultativement substitué avec un ou plusieurs atomes de fluor, un groupe alkoxy en $C_1$ à $C_4$ ;
X représente un atome d'halogène ;
B est choisi entre des groupes :

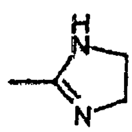, 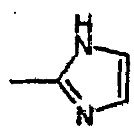, 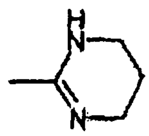, 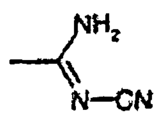, 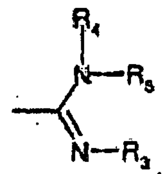, 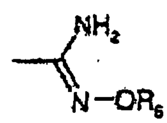,

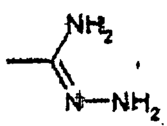, 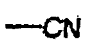, 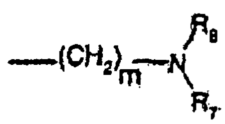, 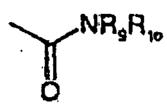 et 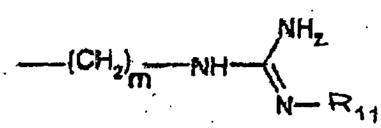

dans lesquels $R_3$, $R_4$, $R_5$, $R_6$, $R_7$, $R_8$, $R_9$ et $R_{10}$, qui sont identiques ou différents, sont choisis entre l'hydrogène et un groupe alkyle en $C_1$ à $C_4$ ; $R_{11}$ représente l'hydrogène, un groupe alkyle en $C_1$ à $C_4$ ou hydroxy, et m est égal à 0, 1 ou 2 ; ou ses sels pharmaceutiquement acceptables.

2. Composé suivant la revendication 1, dans lequel $R_3$, $R_4$, $R_5$, $R_6$, $R_7$, $R_8$, $R_9$, $R_{10}$ et $R_{11}$ représentent, indépendamment les uns des autres, l'hydrogène, un groupe méthyle ou éthyle.

3. Composé suivant la revendication 1 ou 2, dans lequel

n est égal à 3 ;
c est égal à 1 ;
A représente une liaison ou un groupe vinylène ;
$R_1$ et $R_2$, qui sont identiques ou différents, sont choisis entre l'hydrogène, des groupes méthyle, méthoxy et trifluorométhyle ;
X représente un groupe chloro ou bromo ;
B est choisi entre des groupes :

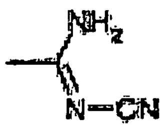, 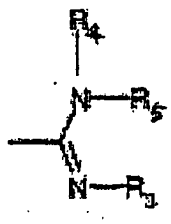, 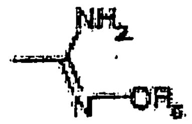, 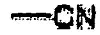,

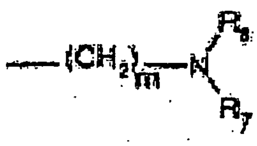, 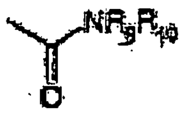 et 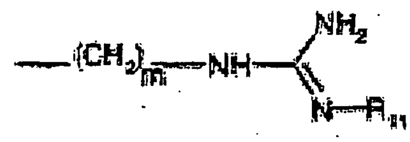

dans lesquels $R_3$, $R_4$, $R_5$, $R_7$, $R_8$, $R_9$, $R_{10}$ et $R_{11}$, qui sont identiques ou différents, sont choisis entre l'hydrogène et un groupe méthyle ; $R_6$ représente un atome d'hydrogène ; et m est égal à 0 ou 1 ;
ou ses sels pharmaceutiquement acceptables.

**4.** Composé choisi dans le groupe consistant en :

3- [1-méthyl-4[1-méthyl-4[1-méthyl-4[4-(2-chloroéthylsulfinyl)phényl-1-carboxamido]pyrrole-2-carboxamido] pyrrole-2-carboxamido]-pyrrole-2-carboxamido]propionamidine ;
3-[1-méthyl-4[1-méthyl-4[1-méthyl-4[4-(2-chloroéthylsulfinyl)phényl-1-carboxamido]pyrrole-2-carboxamido] pyrrole-2-carboxamido]-pyrrole-2-carboxamido]propion-N-méthylamidine ;
3- [1-méthyl-4[1-méthyl-4[1-méthyl-4[4- (2-chloroéthylsulfinyl)phényl-1-carboxamido]pyrrole-2-carboxamido] pyrrole-2-carboxamido]-pyrrole-2-carboxamido]propion-N,N'-diméthylamidine ;
3- [1-méthyl-4[1-méthyl-4[1-méthyl-4[4- (2-chloroéthylsulfinyl)phényl-1-carboxamido]pyrrole-2-carboxamido] pyrrole-2-carboxamido]-pyrrole-2-carboxamido]propion-N,N',N'-triméthylamidine ;
3- [1-méthyl-4[1-méthyl-4[1-méthyl-4[4- (2-chloroéthylsulfinyl)phényl-1-carboxamido]pyrrole-2-carboxamido] pyrrole-2-carboxamido]-pyrrole-2-carboxamido]propion-N-cyanamidine ;
3- [1-méthyl-4[1-méthyl-4[1-méthyl-4[4-(2-chloroéthylsulfinyl)phényl-1-carboxamido]pyrrole-2-carboxamido] pyrrole-2-carboxamido]-pyrrole-2-carboxamido]propionamidoxime ;
3- [1-méthyl-4[1-méthyl-4[1-mé-thyl-4[4-(2-chloroéthylsulfinyl)phényl-1-carboxamido]pyrrole-2-carboxamido] pyrrole-2-carboxamido]-pyrrole-2-carboxamido]propionamide ;
3-[1-méthyl-4[1-méthyl-4[1-méthyl-4[4-(2-chloroéthylsulfinyl)phényl-1-carboxamido]pyrrole-2-carboxamido] pyrrole-2-carboxamido]-pyrrole-2-carboxamido]propion-N-méthylamide ;
3-[1-méthyl-4[1-méthyl-4[1-méthyl-4[4-(2-chloroéthylsulfinyl)phényl-1-carboxamido]pyrrole-2-carboxamido] pyrrole-2-carboxamido]-pyrrole-2-carboxamido]propionitrile ;
2- [1-méthyl-4[1-méthyl-4[1-méthyl-4[4- (2-chloroéthylsulfinyl)phényl-1-carboxamido]pyrrole-2-carboxamido] pyrrole-2-carboxamido]-pyrrole-2-carboxamido]éthylguanidine ;
3- [1-méthyl-4[1-méthyl-4[1-méthyl-4[4- (2-chloroéthylsulfinyl)phényl-1-carboxamido]pyrrole-2-carboxamido] pyrrole-2-carboxamido]-pyrrole-2-carboxamido]propion-N,N-diméthylamine ;
3- [1-méthyl-4[1-méthyl-4[1-méthyl-4[4- (2-bromoéthylsulfinyl)phényl-1-carboxamido]pyrrole-2-carboxamido] pyrrole-2-carboxamido]-pyrrole-2-carboxamido]propionamidine ;
3-[1-méthyl-4[1-méthyl-4[1-méthyl-4[3-méthyl-4-(2-chloroéthyl-sulfinyl)phényl-1-carboxamido]pyrrole-2-carboxamido]-pyrrole-2-carboxamido]-pyrrole-2-carboxamido]propionamidine ;
3-[1-méthyl-4[1-méthyl-4[1-méthyl-4[4-(2-chloroéthylsulfinyl)-cinnamoyl-1-carboxamido]pyrrole-2-carboxamido]pyrrole-2-carboxamido]-pyrrole-2-carboxamido]propionamidine ;
3-[1-méthyl-4[1-méthyl-4[1-méthyl-4[4-(2-chloroéthylsulfinyl)cinnamoyl-1-carboxamido]pyrrole-2-carboxamido]pyrrole-2-carboxamido]-pyrrole-2-carboxamido]propion-N-méthylamidine ;
3-[1-méthyl-4[1-méthyl-4[1-méthyl-4[4-(2-chloroéthylsulfinyl)cinnamoyl-1-carboxamido]pyrrole-2-carboxamido]pyrrole-2-carboxamido]-pyrrole-2-carboxamido]-propion-N,N'-diméthylamidine ;
3-[1-méthyl-4[1-méthyl-4[1-méthyl-4[4-(2-chloroéthylsulfinyl)cinnamoyl-1-carboxamido]pyrrole-2-carboxamido]pyrrole-2-carboxamido]-pyrrole-2-carboxamido]-propion-N,N,-diméthylamidine ;
3-[1-méthyl-4[1-méthyl-4[1-méthyl-4[4-(2-chloroéthylsulfinyl)cinnamoyl-1-carboxamido]pyrrole-2-carboxamido]pyrrole-2-carboxamido]-pyrrole-2-carboxamido]propion-N-cyanamidine ;

3-[1-méthyl-4[1-méthyl-4[1-méthyl-4[4-(2-chloroéthylsulfinyl)cinnamoyl-1-carboxamido]pyrrole-2-carboxamido]pyrrole-2-carboxamido]-pyrrole-2-carboxamido]-propionamidoxime ;
3-[1-méthyl-4[1-méthyl-4[1-méthyl-4[4-(2-chloroéthylsulfinyl)cinnamoyl-1-carboxamido]pyrrole-2-carboxamido]pyrrole-2-carboxamido]-pyrrole-2-carboxamido]-propionamide ;
3-[1-méthyl-4[1-méthyl-4[1-méthyl-4[4-(2-chloroéthyl-sulfinyl)cinnamoyl-1-carboxamido]pyrrole-2-carboxamido]pyrrole-2-carboxamido]-pyrrole-2-carboxamido]-propionamide ;
3-[1-méthyl-4[1-méthyl-4[1-méthyl-4[4-(2-chloroéthylsulfinyl)cinnamoyl-1-carboxamido]pyrrole-2-carboxamido]pyrrole-2-carboxamido]-pyrrole-2-carboxamido]-propionitrile ;
2-[1-méthyl-4[1-méthyl-4[1-méthyl-4[4-(2-chloroéthylsulfinyl)phényl-1-carboxamido]pyrrole-2-carboxamido] pyrrole-2-carboxamido]-pyrrole-2-carboxamido]-éthylguanidine ;
3-[1-méthyl-4[1-méthyl-4[1-méthyl-4[4-(2-chloroéthylsulfinyl)cinnamoyl-1-carboxamido]pyrrole-2-carboxamido]-pyrrole-2-carboxamido]-pyrrole-2-carboxamido]propion-N,N,N'-triméthylamidine ;
3-[1-méthyl-4[1-méthyl-4[1-méthyl-4[4-(2-chloroéthyl-sulfonyl)phényl-1-carboxamido]pyrrole-2-carboxamido]pyrrole-2-carboxamido]-pyrrole-2-carboxamido]-propionamidine ;
3- [1-méthyl-4 [1-méthyl-4 [1-méthyl-4[4- (2-chloroéthylsulfonyl)phényl-1-carboxamido]pyrrole-2-carboxamido]pyrrole-2-carboxamido]-pyrrole-2-carboxamido]-propion-N-méthylamidine ;
3-[1-méthyl-4[1-méthyl-4[1-méthyl-4[4-(2-chloroéthyl-sulfonyl)phényl-1-carboxamido]pyrrole-2-carboxamido]-pyrrole-2-carboxamido]-pyrrole-2-carboxamido]propion-N,N'-diméthylamidine ;
3-[1-méthyl-4[1-méthyl-4[1-méthyl-4[4-(2-chloroéthylsulfonyl)phényl-1-carboxamido]pyrrole-2-carboxamido]-pyrrole-2-carboxamido]-pyrrole-2-carboxamido]propion-N-cyanamidine ;
3-[1-méthyl-4[1-méthyl-4[1-méthyl-4[4-(2-chloroéthylsulfonyl)phényl-1-carboxamido]pyrrole-2-carboxamido]-pyrrole-2-carboxamido]-pyrrole-2-carboxamido]-propionamidoxime ;
3-[1-méthyl-4[1-méthyl-4[1-méthyl-4[4-(2-chloroéthylsulfonyl)phényl-1-carboxamido]pyrrole-2-carboxamido]-pyrrole-2-carboxamido]-pyrrole-2-carboxamido]propionamide ; 3-[1-méthyl-4[1-méthyl-4[1-méthyl-4 [4-(2-chloroéthylsulfinyl)phényl-1-carboxamido]pyrrole-2-carboxamido]-pyrrole-2-carboxamido]-pyrrole-2-carboxamido]propionitrile ;
2-[1-méthyl-4[1-méthyl-4[1-méthyl-4[4-(2-chlorcéthylsulfinyl)phényl-1-carboxamido]pyrrole-2-carboxamido]-pyrrole-2-carboxamido]-pyrrole-2-carboxamido]éthylguanidine ;
3- [1-méthyl-4[1-méthyl-4[1-méthyl-4 [4- (2-chloroéthylsulfonyl)-cinnamoyl-1-carboxamido]pyrrole-2-carboxamido]pyrrole-2-carboxamido]-pyrrole-2-carboxamido]propionamidine ;
3- [1-méthyl-4 [1-méthyl-4[1-méthyl-4 [4-(2-chloroéthylsulfonyl)cinnamoyl-1-carboxamido]pyrrole-2-carboxamido]-pyrrole-2-carboxamido]-pyrrole-2-carboxamido]propion-N-méthylamidine ;
3-[1-méthyl-4[1-méthyl-4[1-méthyl-4[4-(2-chloroéthylsulfonyl)cinnamoyl-1-carboxamido]pyrrole-2-carboxamido]-pyrrole-2-carboxamido]-pyrrole-2-carboxamido]propion-N,N'-diméthylamidine ;
3-[1-méthyl-4[1-méthyl-4[1-méthyl-4[4-(2-chloroéthylsulfonyl)cinnamoyl-1-carboxamido]pyrrole-2-carboxamido]-pyrrole-2-carboxamido]-pyrrole-2-carboxamido]propion-N-cyanamidine ;
3-[1-méthyl-4[1-méthyl-4[1-méthyl-4[4-(2-chloroéthylsulfonyl)cinnamoyl-1-carboxamido]pyrrole-2-carboxamido]-pyrrole-2-carboxamidol-pyrrole-2-carboxamido]-propionamidoxime ;
3-[1-méthyl-4[1-méthyl-4[1-méthyl-4[4-(2-chloroéthylsulfonyl)cinnamoyl-1-carboxamido]pyrrole-2-carboxamido]-pyrrole-2-carboxamido]-pyrrole-2-carboxamido]propionamide ; 3 - [1-méthyl-4 [1-méthyl-4[1-méthyl-4 [4-(2-chloroéthylsulfonyl)-cinnamoyl-1-carboxamido]pyrrole-2-carboxamido]pyrrole-2-carboxamido]-pyrrole-2-carboxamido]propionitrile ;
2- [1-méthyl-4 [1-méthyl-4[1-méthyl-4[4-(2-chloroéthylsulfonyl)-cinnamoyl-1-carboxamido]pyrrole-2-carboxamido]pyrrole-2-carboxamido]-pyrrole-2-carboxamido]éthylguanidine ; et ses sels pharmaceutiquement acceptables.

**5.** Procédé pour la préparation des composés de formule (I) et leurs sels pharmaceutiquement acceptables, procédé qui comprend :

(a) lorsque B est autre que des groupes

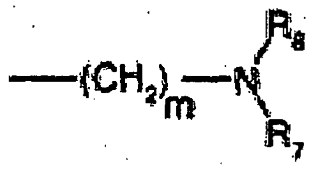

et

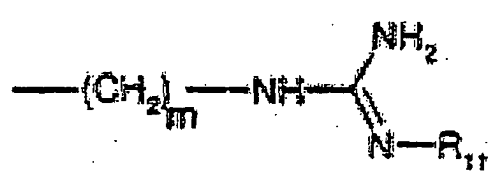

la réaction d'un composé de formule :

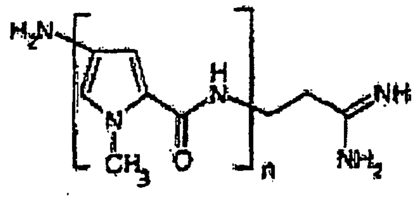

(II)

avec un composé de formule :

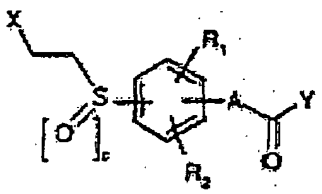

(III)

formules dans lesquelles n, c, $R_1$, $R_2$, X et A répondent aux définitions figurant dans la revendication 1, et Y représente un groupe hydroxy ou un groupe portant convenable ;

de manière à obtenir un composé de formule :

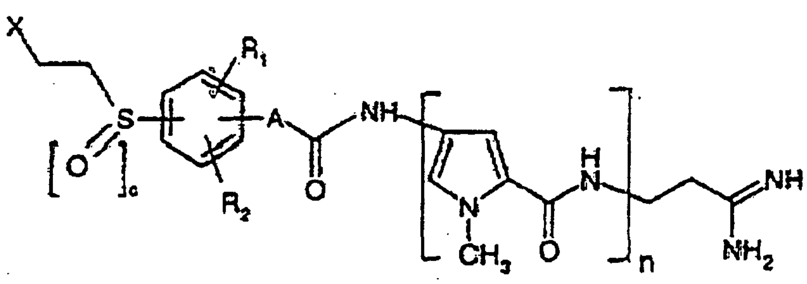

(Ia)

et ensuite, facultativement, la réaction d'un composé de formule (Ia) avec :

(i) un composé de formule $H_2N-(CH_2)_r-NH_2$ dans laquelle r est égal à 2 ou 3, de manière à obtenir un composé de formule (I) dans lequel B représente un groupe :

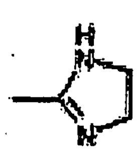

ou

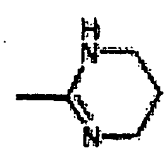

(ii) un composé de formule $H_2N-CH_2-CHO$, de manière à obtenir un composé de formule (I) dans lequel B représente un groupe :

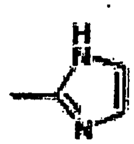

(iii) un composé de formule $H_2N-CN$, de manière à obtenir un composé de formule (I) dans lequel B représente un groupe :

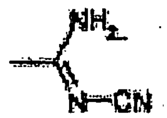

(iv) un composé de formule $H_2N-OR_6$, de manière à obtenir un composé de formule (I) dans lequel B représente un groupe :

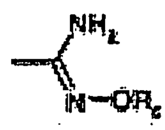

(v) un composé de formule $N_2N-NH_2$, de manière à obtenir un composé de formule (I) dans lequel B représente un groupe :

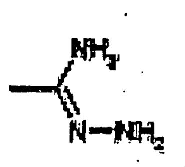

(vi) un composé de formule $HNR_4R_5$, de manière à obtenir un composé de formule (I) dans lequel B représente un groupe :

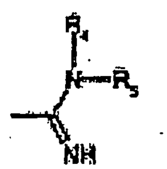

et ensuite, facultativement, avec un composé de formule $H_2NR_3$, de manière à obtenir un composé de formule (I) dans lequel B représente un groupe :

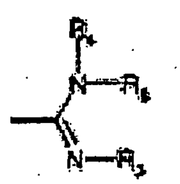

(vii) de l'anhydride succinique, de manière à obtenir un composé de formule (I) dans laquelle B représente un groupe -C≡ N ;

(viii) de l'eau dans un milieu alcalin, de manière à obtenir un composé de formule (I) dans laquelle B représente un groupe $-CONR_9R_{10}$ dans lequel $R_9$ et $R_{10}$ représentent l'un et l'autre un atome d'hydrogène ;

(ix) un composé de formule $HNR_9R_{10}$, de manière à obtenir un composé de formule (I) dans laquelle B représente un groupe :

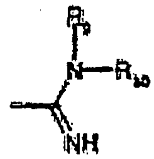

et ensuite avec de l'eau dans un milieu alcalin, de manière à obtenir un composé de formule (I) dans laquelle B représente un groupe -CONR$_9$R$_{10}$, dans lequel R$_9$ et R$_{10}$ sont tels que définis dans la revendication 1 ; ou

(b) lorsque B est autre qu'un groupe :

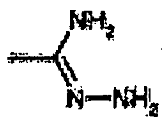

la réaction d'un composé de formule :

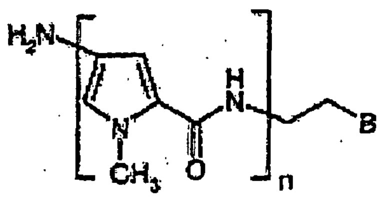

(IV)

avec un composé de formule :

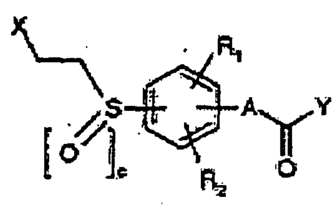

(III)

formules dans lesquelles n, c, B, R$_1$, R$_2$, X, Y et A répondent aux définitions précitées ;
de manière à obtenir le composé correspondant de formule (I) :
et, si cela est désiré, la conversion du composé de formule (I) en un de ses sels pharmaceutiquement acceptables.

**6.** Procédé suivant la revendication 5, dans lequel dans les composés de formule (III), Y représente un groupe hydroxy ou un groupe choisi entre des groupes chloro, 2,4,5-trichlorophénoxy, 2,4-dinitrophénoxy, succinimido-N-oxy et imidazolyle.

**7.** Composition pharmaceutique comprenant un ou plusieurs supports et/ou diluants pharmaceutiquement acceptables et, comme principe actif, un composé tel que défini dans la revendication 1.

**8.** Composé tel que défini dans la revendication 1, destiné. à être utilisé dans une méthode de traitement de l'organisme humain ou animal par thérapie.

**9.** Composé tel que défini dans la revendication 8, destiné à être utilisé comme agent antitumoral.

**10.** Utilisation d'un composé tel que défini dans la revendication 1 dans la production d'un médicament destiné à être utilisé comme agent antitumoral.